(19) Europäisches Patentamt
European Patent Office
Office européen des brevets

(11) Numéro de publication: **0 244 320 B1**

# (12) FASCICULE DE BREVET EUROPEEN

(45) Date de publication de fascicule du brevet: **08.07.92**

(21) Numéro de dépôt: **87400980.6**

(22) Date de dépôt: **29.04.87**

(51) Int. Cl.⁵: **C07D 471/06**, C07D 471/22, C07H 1/00, //(C07D471/06, 221:00,221:00),(C07D471/22, 257:00,221:00,221:00,221:00, 221:00)

## (54) Procédé de photoclivage des acides nucléiques à l'aide de dications 2,7-diazapyrénium, nouveaux dérivés diazapyréniques.

(30) Priorité: **29.04.86 FR 8606212**

(43) Date de publication de la demande:
**04.11.87 Bulletin 87/45**

(45) Mention de la délivrance du brevet:
**08.07.92 Bulletin 92/28**

(84) Etats contractants désignés:
**AT BE CH DE ES FR GB GR IT LI LU NL SE**

(56) Documents cités:
**US-A- 3 652 149**

**JOURNAL OF THE AMERICAN CHEMICAL SOCIETY, vol. 103, 1981, pages 3582-3584, American Chemical Society, Columbus, Ohio, US; K.A. REICH et al.: "Cleavage of DNA by the 1,10-phenanthroline-copper ion complex. Superoxide mediates the reaction dependent on NADH and hydrogen peroxide"**

(73) Titulaire: **CIS BIO INTERNATIONAL**
**RN 306**
**F-91000 Saclay(FR)**

(72) Inventeur: **Lehn, Jean-Marie**
**21, rue d'Oslo**
**F-67000 Strasbourg(FR)**
Inventeur: **Blacker, John**
**ICI Biological Products, PO Box 1**
**Billingham, Cleveland TS 23 1 LB(GB)**
Inventeur: **Jazwinski, Jaroslaw**
**Ul Siemionowicza 22**
**Warszawa 01-481(PL)**

(74) Mandataire: **Gillard, Marie-Louise et al**
**Cabinet Beau de Loménie 55, Rue d'Amsterdam**
**F-75008 Paris(FR)**

EP 0 244 320 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 75, 1971, page 378, résumé no. 157073x, Columbus, Ohio, US; & ZA-A-70 06 357 (IMPERIAL CHEMICAL INDUSTRIES LTD) 30-03-1971

JOURNAL OF THE CHEMICAL SOCIETY, CHEMICAL COMMUNICATIONS, 1986, pages 1035-1037, London, GB; A.J. BLACKER et al.: "Photochemical cleavage of DNA by 2,7-diazapyrenium cations"

Science, 1985, 227, pages 849 and following

## Description

La présente invention concerne un procédé de photoclivage des acides nucléiques à l'aide de dications 2,7-diazapyrénium $DAP^{2+}$ ou de leurs espèces dimères bis-$DAP^{4+}$.

Elle a également pour objet des nouveaux dérivés diazapyréniques; il s'agit de sels qui, en solution, libèrent des dications $DAP^{2+}$ ou des tétracations bis-$DAP^{4+}$.

Enfin, les dications 2,7-diazapyrénium couplés à des oligonucléotides peuvent constituer des réactifs de photoexcision spécifiques pour les acides nucléiques.

De nombreuses recherches ont été entreprises ces dernières années pour trouver des réactifs artificiels capables de cliver les acides nucléiques et plus particulièrement l'acide désoxyribonucléique ou l'acide ribonucléique, communément dénommés ADN et ARN.

La plupart des études ont été réalisées avec des complexes métalliques qui clivent l'ADN par un procédé redox oxygène-dépendant ou par photo-activation.On a également utilisé des colorants du type acridine pour la photodestruction de l'ADN.

Parmi les complexes métalliques qui ont été utilisés, on peut citer notamment les composés portant des groupes EDTA-Fe(II) et les complexes 1,10-phénanthroline-cuivre.

Par exemple, BOUTORIN et al. dans FEBS LETTERS vol. 172, N°1 Juin 1984 décrit des réactifs portant des groupes EDTA-Fe(II) qui conviennent pour le clivage direct des acides nucléiques simples brins.

De même TULLIUS et al. dans SCIENCE vol. 230, 1985 p. 679-681 utilise l'EDTA-Fe(II) pour couper l'ADN.

HERTZBERG et al. [J. Am. Chem. Soc. 1982 104 p. 313-315] préconise l'utilisation de (méthidiumpropyl-EDTA)-Fe(II) comme cofacteur dans la coupure de l'ADN pour un certain nombre d'antibiotiques antitumoraux.

L'utilisation du complexe 1,10-phénanthroline-cuivre pour le clivage de l'ADN est décrite notamment par REICH et al. dans J. Am. Chem. Soc. Vol. 103 No. 12, 1981 et par R. FAGGIANI et al. dans J. Am. Chem. Soc. Vol. 102 p.5419-5421. 1980.

D'autre part, il est connu que les colorants du type acridine endommagent l'ADN. Par exemple FREIFELDER et al. [Biophysical Journal Vol. 1, 1961] ont montré que de l'ADN irradié avec de la lumière visible en présence d'acridine orange se dépolymérise et se coupe.

On notera par ailleurs que les ions de 4,4'-bipyridinium, et en particulier le méthyl-viologène ont été utilisés dans différents procédés redox et photo-redox [AMOUYAL et al. Israel Journal of Chemistry 22, 1982 p. 117-123]. Ces derniers ne peuvent être réalisés qu'en présence d'un photosensibilisateur ou sous une irradiation ultraviolette.

Le méthyl-viologène, qui est le chlorure de 1,1'-diméthyl-4,4'-bipyridinium, couramment dénommé $MV^{2+}$, peut détériorer l'ADN dans ces conditions.

Le brevet US 3 652 149 concerne un dispositif de filtration de la lumière mettant en oeuvre un composé redox capable de former un radical libre stable et coloré par addition d'électrons. Les composés diazapyréniques utilisés comme composés redox selon ce brevet US sont ceux de formule :

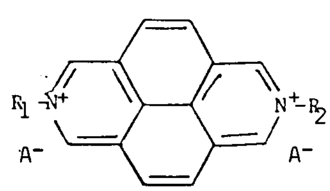

dans laquelle $R_1$ et $R_2$ sont respectivement choisis parmi l'hydrogène, un alkyle, un aralkyle et un aryle et $A^-$ est un anion.

On a maintenant trouvé que l'on peut utiliser des dérivés diazapyréniques et plus particulièrement les cations 2,7-diazapyrénium pour le photoclivage des acides nucléiques à la lumière visible sans qu'il soit nécessaire d'utiliser des photosensibilisateurs.

Ainsi la présente invention concerne un procédé pour le photoclivage des acides nucléiques qui consiste à mettre en contact un acide nucléique avec une solution d'un dérivé du 2,7-diazapyrène tel que défini ci-après et à soumettre le mélange réactionnel obtenu à une irradiation à la lumière visible.

Les dérivés diazapyréniques qui conviennent aux fins de l'invention répondent à la formule (I) ci-après :

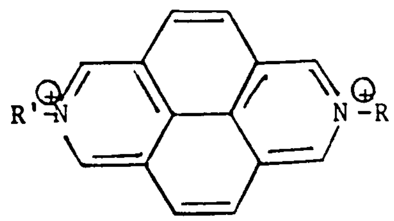

$2X^-$ (I)

dans laquelle :

R' est l'hydrogène ou un groupe alkyle en $C_1$-$C_6$ et R est l'hydrogène ou un groupe alkyle substitué choisi parmi les groupes de formules $-(CH_2)_n$-SH, $-(CH_2)_n$-OH et $-(CH_2)_n$-$NH_2$, dans lesquels n est compris entre 1 et 10, $X^-$ est un anion, avec la condition que R et R' ne soient pas en même temps l'hydrogène,

ou bien R et R' sont identiques et représentent (a) le groupe de formule $-R_1Y$-, dans laquelle $R_1$ est $-(CH_2)_3$- et $Y^-$ l'ion sulfonate, $SO^-_3$, ou (b) le groupe de formule :

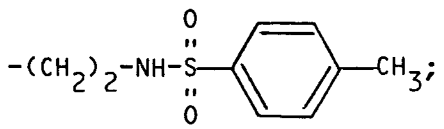

; et

$X^-$ est un anion.

Les composés de formule I ci-dessus dans laquelle R et R' sont identiques et représentent l'hydrogène ou un groupe méthyle, sont des composés connus et décrits par Hunïg et al. [Angew. Chem. 1980 N°19 (1968)].

On peut également utiliser dans le procédé de l'invention les dérivés bis-diazapyréniques qui répondent à la formule générale

(II) :

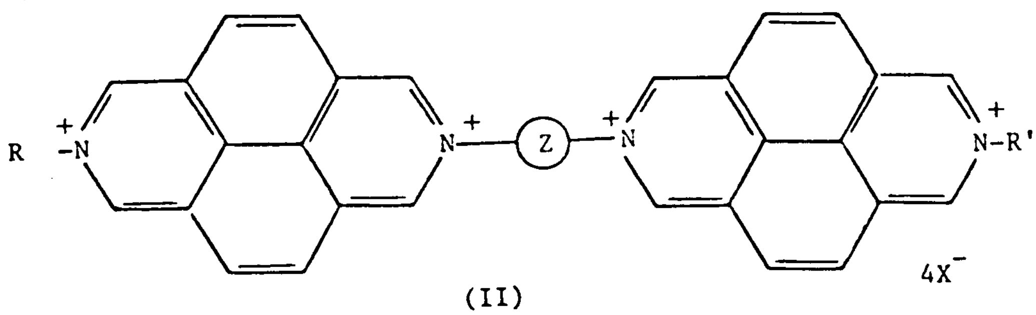

$4X^-$

(II)

dans laquelle R et R' sont identiques ou différents et représentent chacun l'hydrogène ou un groupe alkyle en $C_1$-$C_6$ ; $X^-$ est un anion et Z est choisi parmi les radicaux divalents de formules :

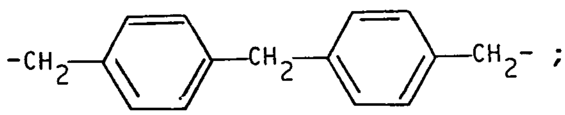

(III)

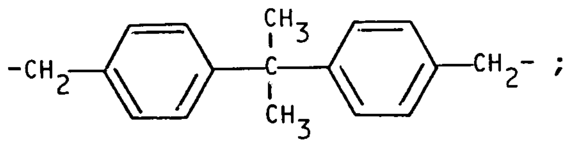

et (IV)

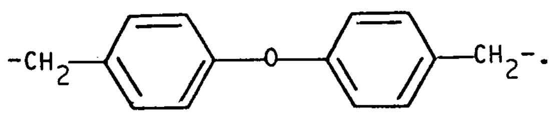

(V)

Dans les formules (I) et (II) ci-dessus, les radicaux hydrocarbonés aliphatiques ont de préférence 1 à 10 atomes de carbone. Les groupes alkyle linéaires ou ramifiés ayant 1 à 6 atomes de carbone sont particulièrement préférés.

Les dérivés diazapyréniques de formule (I) et (II) peuvent tous être obtenus à partir du 2,7-diazapyrène ou DAP par l'un des procédés ci-après. Par commodité, on désignera dans la suite de la présente description les dérivés diazapyréniques de formule (I) par l'expression "dérivés $DAP^{2+}$" et ceux de formule (II) par l'expression "dérivés bis-$DAP^{4+}$".

Synthèse des dérivés $DAP^{2+}$

Procédé A

$$DAP + RX_1 \rightarrow DAP^{+}\text{-}R\ X_1^{-}$$

$$DAP^{+}\text{-}R\ X_1^{-} + R'X_2 \longrightarrow R\text{-}^{+}DAP^{+}\text{-}R'\ X_1^{-}\ X_2^{-}$$

$X_1$ et $X_2$, identiques ou différents, ont la même signification que X.

Ce procédé comporte essentiellement deux étapes de N-substitution du DAP, respectivement par les radicaux R et R', le composé obtenu peut ensuite être éventuellement transformé, par échange d'ions,en le composé correspondant dans lequel $X_1$ et $X_2$ sont identiques. On notera que lorsque R et R' sont identiques, les deux étapes de substitution sont réalisées simultanément. Ces étapes sont réalisées dans un solvant approprié, par exemple le diméthylformamide (DMF), le chloroforme ou l'acétonitrile. Le DMF convient lorsqu'on souhaite obtenir les deux substitutions simultanément ($R = R'CH_3$ par exemple), la réaction étant effectuée à température ambiante, tandis qu'il convient d'utiliser le chloroforme pour la première étape de substitution et l'acétonitrile pour la seconde ($R \neq R'$), auquel cas on opère avantageusement à la température ambiante pour la première étape de substitution et au reflux pour la seconde.

Ce procédé convient notamment pour l'obtention des dérivés $DAP^{2+}$ dans lesquels R et R' sont un radical hydrocarboné aliphatique tel que défini ci-dessus et en particulier pour l'obtention du dichlorure de N,N'-diméthyl-2,7-diazapyrénium dénommé ci-après $MDAP^{2+}(Cl^{-})_2$ qui est illustrée par le schéma ci-après :

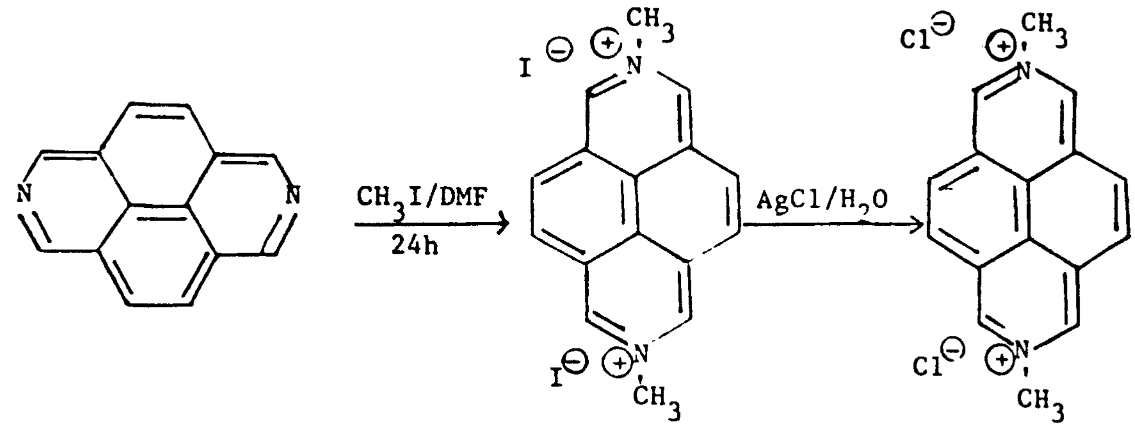


DAP $MDAP^{2+} (I^-)_2$ $MDAP^{2+} (Cl^-)_2$

Procédé B

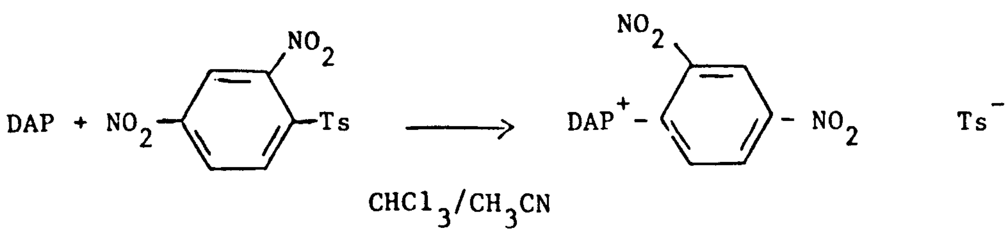


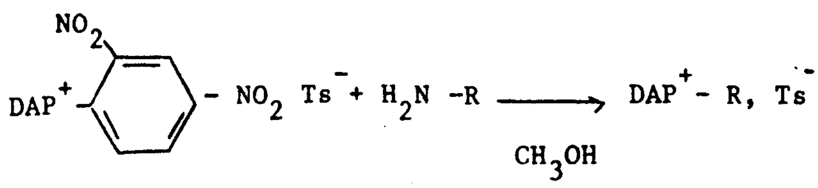


$DAP^+ - R\ Ts^- + R'X_1 \longrightarrow R' - {}^+DAP^+ - R\ \ Ts^-, X^-$

DMF

échange d'ions

$R' - {}^+DAP^+ - R,\ 2Cl^-$

avec $Ts^-$ = anion, par exemple le tosylate

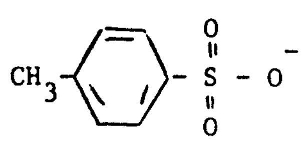

On notera que $Ts^-$ peut être remplacé par un autre anion.

Ce procédé convient pour l'obtention des dérivés $DAP^{2+}$ dans lesquels le radical R est un radical hydrocarboné aliphatique substitué par un groupe hydroxy, en particulier un groupe hydroxyalkyle et R' est un groupe alkyle, par exemple le méthyle. Ce procédé consiste à fabriquer d'abord un sel de 2-dinitrophényl-2,7-diazapyrénium par exemple le tosylate, à titre d'intermédiaire de synthèse par réaction du DAP avec un sel de 2,4-dinitrophényle, tel que le tosylate, dans un solvant approprié, par exemple un mélange chloroforme/méthanol à reflux, puis à faire réagir cet intermédiaire avec une amine substituée par un radical R ; la seconde étape est une étape de N-substitution avec un halogénure de formule R'X comme dans le procédé A.

Procédé C

Ce procédé convient pour l'obtention des dérivés $DAP^{2+}$ dans lesquels R' est un groupe alkyle par exemple le méthyle et R est un reste hydrocarboné aliphatique substitué par un groupe amino. Ce procédé est semblable au procédé B ci-dessus à la seule différence que l'on utilise comme amine une diamine dont l'une des fonctions amine est protégée, laquelle est ensuite déprotégée avant la seconde étape de substitution. Ce procédé peut être schématisé comme suit :

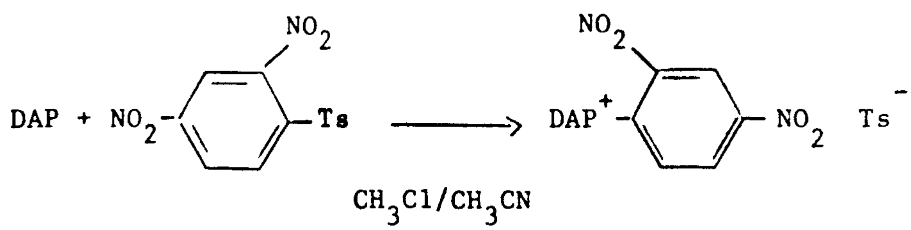


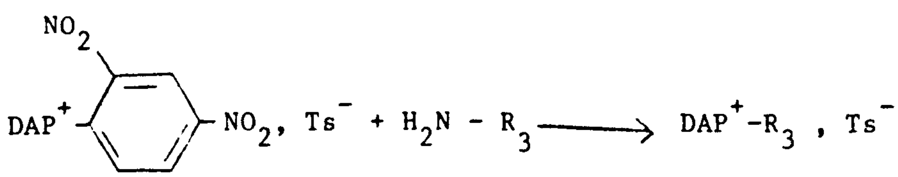

$$DAP^+ - R_3\ ,Ts^- \xrightarrow{\text{déprotection}} DAP^+ - Alk - NH_2\ ,Ts^-$$

$$DAP^+\text{-}Alk\text{-}NH_2\ ,\ Ts^- + R'X \longrightarrow R' - {}^+DAP^+ - Alk - NH_2,\ Ts^-X^-$$

avec $R_3$ = Alk - $NH_2$ (protégé).

Le tosylate de 2-dinitrophényl-2,7-diazapyrénium est un nouveau composé utile comme intermédiaire de synthèse ; il constitue un autre objet de l'invention.

Procédé D

Ce procédé convient pour l'obtention du dérivé $DAP^{2+}$ dans lequel R et R' sont identiques et représentent un radical $R_1Y^-$ tel que défini précédemment ;

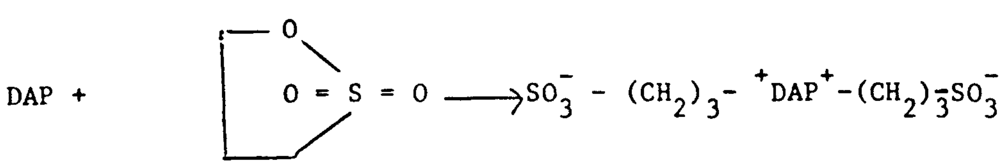

Ce procédé consiste à faire réagir ensemble du DAP partiellement dissous dans de l'acétone anhydre et du propansultone à la température de reflux.

Procédé E

Ce procédé convient pour l'obtention du dérivé DAP$^{2+}$ dans lequel R et R' sont identiques et représentent le groupe de formule :

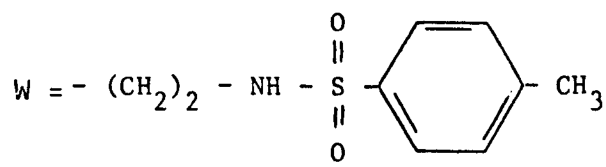

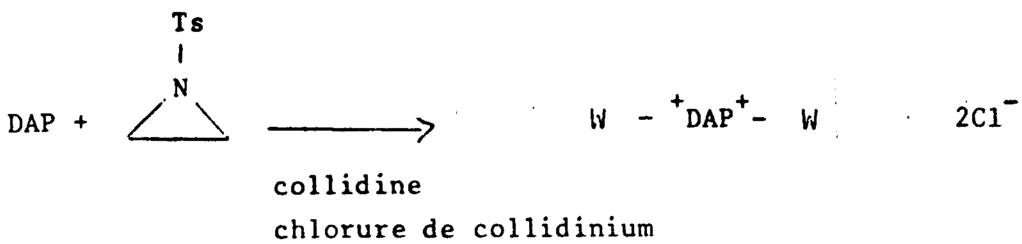


Ce procédé consiste donc à faire réagir le DAP avec l'aziridine en présence de collidine et de chlorure de collidinium.

Synthèse des dérivés bis-DAP$^{4+}$

Le procédé d'obtention de ces dérivés bis-DAP$^{4+}$ consiste essentiellement à faire réagir deux molécules de DAP avec une molécule d'un dihalogénure de formule $X_1$-Ⓩ-$X_1$ dans laquelle $X_1$, $X_2$ et Ⓩ sont tels que définis ci-dessus et à procéder ensuite à la N-substitution des azotes restants dans les molécules de DAP. Chaque étape est avantageusement réalisée dans l'acétonitrile à la température de reflux.

Ce procédé peut être schématisé comme suit :

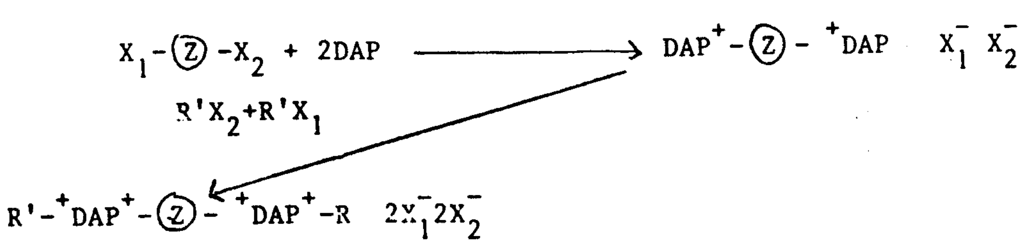


Les dérivés diazapyréniques selon l'invention sont des composés fluorescents.

Le diazapyrène (DAP) utilisé comme produit de départ dans les synthèses ci-dessus peut être obtenu à partir de l'anhydride 1,4,5,8-naphtalènetétracarboxylique de formule VI disponible dans le commerce selon le schéma réactionnel ci-après :

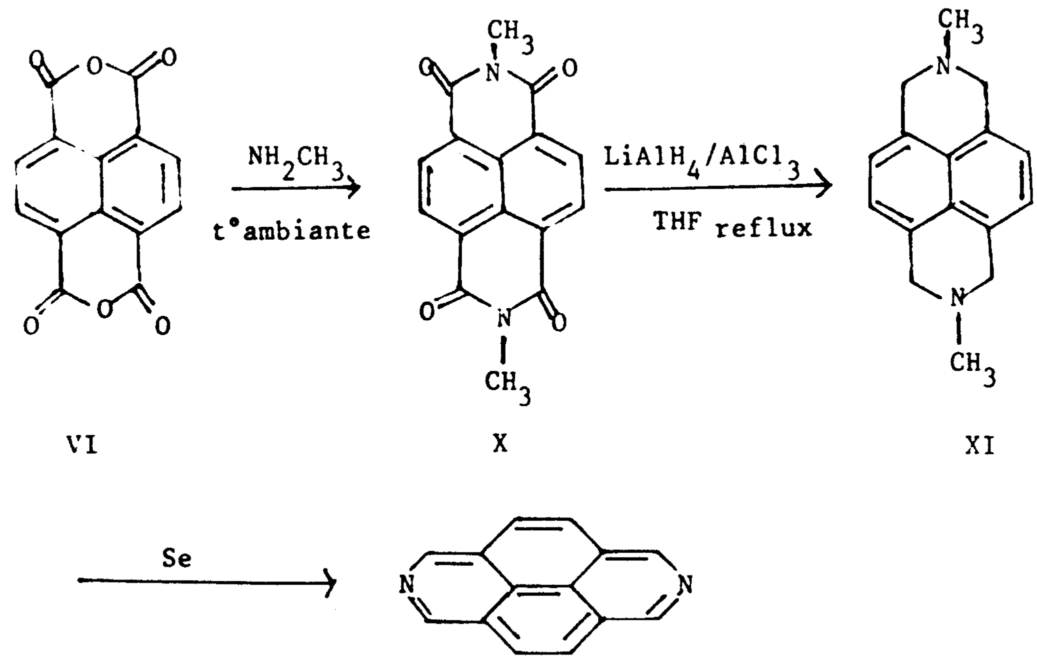


VI X XI

DAP

Selon une adaptation du procédé de Hünig et al. Angew. Chem. 180 (1968) n°19, l'anhydride 1,4,6,8-naphtalènetétracarboxylique de formule VI est tout d'abord transformé en le bis (N-méthylimide) correspondant de formule X par réaction avec de la méthylamine à la température ambiante. L'imide X est ensuite traité avec un mélange $LiAlH_4/AlCl_3$ dans le tétrahydrofuranne à reflux pour former le composé de formule XI , lequel est ensuite mis à réagir pendant 4 heures environ en présence de sélénium à une température comprise entre 265 et 300 ° C pour obtenir le DAP.

Les composés particulièrement appropriés aux fins de l'invention sont :

1) les composés de formule (I) dans laquelle R et R' sont un groupe méthyle ou dérivé $MDAP^{2+}$ ;

2) les composés de formule (I) dans laquelle l'un des substituants R ou R' est un alkyle inférieur, par exemple le méthyle et l'autre est un groupe alkyle substitué par un groupe hydroxy, thio ou un groupe amino, à savoir les groupes de formules $-(CH_2)_n-OH$ , $-(CH_2)_n-SH$,$-(CH_2)_n-NH_2$ dans lesquels n est de préférence compris entre 1 et 10 ;

3) les composés de formule (II) dans laquelle Ⓩ répond à l'une des formules III à V et R et R' sont un groupe alkyle inférieur, par exemple le méthyle.

Les dérivés diazapyréniques de formules (I) ou (II) comportant un ou deux dications 2,7-diazapyrénium ont une structure plane. Ces composés, grâce à cette structure plane interagissent avec les nucléotides de l'ADN ou de l'ARN de la même manière que les médicaments d'intercalation, tels que l'actinomycine, la bléomycine. De plus, sous l'action d'un irridiation à la lumière visible, les dérivés selon l'invention peuvent couper l'ADN ou l'ARN en plusieurs brins.

Il y a lieu de préciser que les dérivés de formule (II) ou dérivés bis-$DAP^{4+}$ sont plus réactifs que les dérivés de formule (I) ou dérivés $DAP^{2+}$ du fait de la présence dans leur molécule de deux cations 2,7-diazapyrénium. Ils présentent d'autre part une sélectivité et une stabilité plus grandes lors du photoclivage.

D'autre part, les dérivés de formule (I) dans lesquels l'un des substituants R ou R' est un radical aliphatique hydrocarboné substitué par un groupe hydroxy ou amino peuvent être couplés par liaison covalente à un oligonucléotide ou un oligodésoxynucléotide comportant un enchaînement de nucléotides naturels, éventuellement modifiés ou synthétisés.

Ce couplage peut être réalisé par des procédés connus de l'homme de l'art. Par exemple on peut utiliser les procédés de synthèse décrits dans le brevet FR 83 01 223 cité dans la présente demande à titre de référence. Ces procédés consistent entre autre à coupler l'hydroxyle du dérivé $DAP^{2+}$ avec un oligonucléotide 3'-phosphodiester ou 5'-phosphodiester , avec un oligonucléotide-3',5'-bis(phosphodiester), ou avec un oligodésoxynucléotide 2'-phosohodiester, les oligonucléotides ou oligodésoxynucléotides étant correctement protégés. Ainsi, on peut obtenir des produits de couplage oligonucléotide (ou oligodésoxynucléotide) -$DAP^{2+}$ ou $DAP^{2+}$-oligonucléotide (ou oligodésoxynucléotide) -$DAP^{2+}$ qui peuvent constituer des réactifs de photoexcision très sélectifs.

La chaîne d'oligonucléotide ou d'oligodésoxynucléotide du produit de couplage peut se lier de façon sélective à toute séquence complémentaire. Grâce à la présence d'au moins un dication 2,7-diazapyrénium dans ce produit de couplage, on peut aisément couper de façon sélective de l'ADN ou de l'ARN en des sites particuliers, dont la position est déterminée par l'oligonucléotide qui se lie à la séquence complémentaire.

On notera que le produit de couplage oligonucléotide -$DAP^{2+}$ peut ultérieurement être couplé avec un autre réactif, qui peut être un agent de révélation quelconque, par exemple un marqueur, tel que notamment un colorant de type acridine ou un agent intercalant, et fournir un produit de couplage bifonctionnel , l'une des fonctions étant le photoclivage, l'autre fonction étant apportée par le second réactif.

Photoclivage de l'ADN

Le procédé de l'invention va être maintenant décrit en référence au $MDAP^{2+}$ ou à son espèce dimère bis-$MDAP^{4+}$ par simple commodité sans pour autant le limiter à ces seuls dérivés.

Des essais réalisés sur l'ADN dans les conditions indiquées ci-après ont montré que le dication $MDAP^{2+}$ ou son espèce dimère bis-$MDAP^{4+}$ coupent l'ADN sous irradiation à la lumière visible.

Les essais ont été effectués par illumination pendant une heure à 3± 1 °C avec de la lumière visible (projecteur-fente de 250 W muni d'un filtre adéquat pour ne laisser passer que la lumière visible $\lambda > 395$ nm) d'une solution aqueuse contenant de l'ADN circulaire double-brin surenroulé ($2,6\ 10^{-8}$ M), un chlorure de $MDAP^{2+}$ ou bis-$MDAP^{4+}$ ($2,6\ 10^{-4}$ M) dans du tampon tris à pH 7,8. La réaction a été effectuée en présence ou en l'absence d'air (oxygène) ou d'EDTA.

L'ADN circulaire double-brin surenroulé, ci-après dénommé ADNsc a été préparé selon les modes opératoires connus en utilisant des bactéries E.coli infectées avec le plasmide pBR 322 et ultérieurement purifié par électroélution de manière à être pratiquement dépourvu d'ADNsc coupé.

Le plasmide pBR322 donne par incubation avec l'enzyme de restriction ECORI un plasmide linéaire ou DNA linéaire double-brin qui a été utilisé comme marqueur dans l'analyse par électrophorèse réalisée après l'essai de photoclivage ci-dessus.

A titre de comparaison on a réalisé le même essai en remplaçant le dérivé de l'invention par du méthylviologène $MV^{2+}$. On a également soumis au même traitement (illumination à la lumière visible, dans les conditions ci-dessus) une solution d'ADNsc seule, ou une solution d'ADNsc contenant de l'oxygène ou une solution d'ADNsc contenant de l' oxygène et de l'EDTA, à titre de contrôles.

Les résultats obtenus apparaissent sur la figure I annexée qui est une photographie du gel d'électrophorèse des solutions des différents essais et sont commentés ci-après. Sur cette figure, on désigne par :
I l'ADNsc, II l'ADNsc coupé et III l'ADN linéaire ;
les abréviations utilisées désignent respectivement :

M = marqueur
C = contrôle
V = lumière visible
O = oxygène
A = EDTA.

La mesure des densités optiques de chaque bande a donné les proportions suivantes des formes I, II et III en %

| N° de bande | Forme I | Forme II | Forme III |
|---|---|---|---|
| 2 | 93 | 7 | - |
| 6 | 82 | 18 | - |
| 7 | 88 | 12 | - |
| 8 | 86 | 14 | - |
| 9 | - | 73 | 27 |
| 10 | 53 | 47 | - |
| 11 | - | 68 | 32 |
| 12 | - | 77 | 23 |
| 13 | 16 | 72 | 12 |

A) Essais avec le $MDAP^{2+}$

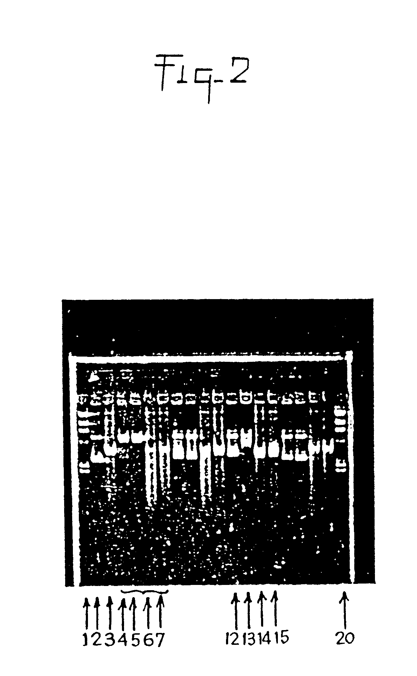

1) Comme le montrent les bandes 9 à 13 sur la figure 1, le $MDAP^{2+}$ clive efficacement l' ADNsc sous irradiation à la lumière visible dans les conditions utilisées, principalement en donnant de l'ADsc coupé par clivage d'un seul brin, mais également de l'ADN linéaire par clivage des deux brins lorsque la réaction se prolonge.

Avec de plus faibles concentrations de $MDAP^{2+}$ il se produit moins de clivage, la transformation en la forme ADNsc coupé étant presque complète à la concentration de 2 $10^{-5}$ M et seulement faible à 2 $10^{-6}$ M et au-dessous.

2) On notera qu'il n'y a pas eu de clivage significatif de l'ADNsc en l'absence de lumière.

3) L'élimination de l'oxygène par dégazage par congélation-décongélation diminue un peu l'efficacité du photoclivage.

4) L'addition d'EDTA n'affecte pas la réaction en présence d'oxygène, mais semble la diminuer lorsque celle-ci est réalisée en l'absence d'oxygène.

5) On notera que la migration de l'ADNsc est quelque peu retardée par le réactif de photoclivage utilisé, ce qui laisse penser que celui-ci s'intercale dans l'ADNsc.

B) Essais avec le bis-$MDAP^{4+}$

1) Dans les mêmes conditions le bis-$MDAP^{4+}$ semble plus efficace que le $MDAP^{2+}$ donnant des petits fragments d'ADN par coupures multiples des deux brins (bandes 14 à 18).

2) La réaction de clivage est tellement efficace que l'exclusion de la lumière devient difficile. Il est néanmoins possible d'observer que la migration de l'ADNsc est notablement retardée, ce qui indique une interaction forte et probablement multiple du Bis-$MDAP^{4+}$ avec le plasmide pBR322.

3) Etant donné l'efficacité de la réaction,il n'est pas possible de noter un effet quelconque de l'oxygène ou de l'EDTA.

L'effet de bis-$MDAP^{4+}$ décroît à des concentrations plus faibles, le clivage étant encore complet à $10^{-5}$ M mais seulement faible à une concentration inférieure.

C) Essais avec $MV^{2+}$ (Bandes 6 à 8) et essais de contrôle (bandes 2 à 5)

Ces essais montrent que, dans les mêmes conditions, il ne se produit pas, ou pratiquement pas, de clivage avec $MV^{2+}$.D'autre part, il n'y a aucune réaction en l'absence de réactif de photoclivage.

Etant donné que d'autres essais ont montré que le $MDAP^{2+}$ photo-oxyde différents substrats sous irradiation à la lumière visible, on peut considérer, sans toutefois vouloir se limiter à une quelconque théorie, que la réaction de clivage a lieu par l'intermédiaire d'une photo-oxydation locale au site d'interaction par l'état excité de l'espèce $MDAP^{2+}$. Le donneur d'électrons peut être le ribose, car on a trouvé que le ribose lui-même photoréduit le $MDAP^{2+}$.

L'effet retardant de l'EDTA en l'absence d'oxygène indiquerait que l'oxydation du donneur extérieur entre en compétition avec l'oxydation du donneur interne.

L'effet produit par l'élimination de l'oxygène, faible mais non négligeable, indique que la réaction de clivage peut aussi faire intervenir le système réactionnel comme avec les complexes métalliques réduits photosensibles. Le cation $DAP^{+}$ produit initialement par photo-réduction pourrait alors réagir avec l'oxygène en générant du superoxide comme cela se produit avec $MV^{+}$ lui-même (Biochim. Biophys. Acta 1973,314 , 372].

D'autres essais ont été réalisés dans les mêmes conditions que ci-dessus avec la proflavine, qui est un colorant de type acridine et on a trouvé que la proflavine est un réactif de photoclivage de l'ADN comparable au Bis-$MDAP^{4+}$ et qu'il donne à la concentration de 2 $10^{-4}$ M un clivage extensif de l'ADN en ADN linéaire. A des concentrations plus faibles allant de 2 $10^{-6}$ M à 2 $10^{-7}$ M, l'efficacité diminue.

On pense que le procédé de photoclivage avec la proflavine implique une photo-oxydation de l'ADN au site d'intercalation avec génération d'un colorant réduit. Les colorants de type acridine sont en effet bien connus pour subir une photoréduction avec une oxydation du substrat.

Les dérivés de l'invention, étant des molécules plates, elles interagissent avec les nucléotides dans l'ADN ou l'ARN de la même manière que les médicaments d'intercalation, tels que l'actinomycine, la bléomycine, etc. On a d'autre part noté que la fluorescence des dérivés disparaît lorsqu'il y a fixation sur l'ADN.Il est donc possible de suivre l'évolution de la réaction de photoclivage par fluorescence.

D'autres essais ont été effectués avec de l'ADN simple brin ou ADNss et sont reportés ci-après.

Les solutions de $MDAP^{2+}$ et bis-$MDAP^{4+}$ à des concentrations allant jusqu'à $4.10^{-4}$ M ont été préparées avec de l'eau distillée deux fois et ajustées à pH 7,6 avec de l'acide chlorhydrique.

De l'ADNsc circulaire et de l'ADNss circulaire ont été obtenus respectivement à partir des plasmides

pBR 322 et M13mp19 et purifiés par des procédés d'électroélution classiques.

Dans des tubes à essai en verre on a mélangé soigneusement :

- le dérivé diazapyrénique $MDAP^{2+}$ ou bis-$MDAP^{4+}$
- l'ADNss ou l'ADNsc
- du tampon Tris pH 7,6

en des quantités appropriées pour obtenir des concentrations en dérivés diazapyréniques de $5.10^{-6}$ M et $10^{-5}$ M. Chaque tube a été maintenu sous une pression constante d'oxygène en utilisant des moyens connus et soumis à une irradiation à la lumière visible (projecteur 250 W ; λ > 395 nm) pendant 1/2 heure, les tubes étant maintenus à 3 ° C dans un bain d'eau glacée.

Après l'irradiation les tubes ont été maintenus à l'obscurité et stockés à 3 ° C. Des échantillons de chaque tube ont été mélangés à du glycérol et soumis à une électrophorèse sur gel d'agarose (Sigma Type II EEO) (6 heures à 120 V et 80 mA). Le gel d'agarose a coloré avec du bromure d'éthidium et photographié. La figure II annexée est une photographie de ce gel sur laquelle les bandes correspondent aux différents essais réalisés,les concentrations étant indiquées ci-après

| N°de bande | ADN | dérivé diazapyrénique | concentration dudit dérivé |
|---|---|---|---|
| 4 | ADNsc | $MDAP^{2+}$ | $10^{-5}$ |
| 5 | " | " | $510^{-6}$ |
| 6 | ADNss | " | $10^{-5}$ |
| 7 | " | " | $510^{-6}$ |
| 12 | ADNsc | bis-$MDAP^{4+}$ | $10^{-5}$ |
| 13 | " | " | $510^{-6}$ |
| 14 | ADNss | " | $10^{-5}$ |
| 15 | " | " | $510^{-6}$ |

les bandes 1 et 20 correspondant aux marqueurs et les bandes 2 et 3 sont représentatives des ADNsc et ADNss soumis à l'irradiation en l'absence de dérivé diazapyrénique ; ces ADN ont été obtenus respectivement par action de l'enzyme $ECoR_1$ et de la nucléase $S_1$ de manière connue.

Cette photographie montre que l'ADNsc et l'ADNss circulaires sont clivés en présence des dérivés diazapyréniques de l'invention.

On notera que les dérivés diazapyréniques mis en oeuvre dans le procédé selon l'invention gardent leur propriété de photoclivage du DNA même lorsqu'ils sont incorporés dans des micelles ou dans des liposomes. On pourra donc utiliser les micelles et les liposomes comme véhicules des dérivés diazapyréniques pour les transporter jusque sur les DNA à couper.

Les dérivés diazapyréniques contenant un radical hydrocarboné à chaîne longue sont particulièrement appropriés pour ce type d'utilisation, puisque le radical hydrocarboné permet une meilleure fixation sur le liposome ou la micelle.

L'invention va être maintenant décrite plus en détail par les exemples ci-après.

EXEMPLE 1 : Préparation du dichlorure de N-méthyl-N'-benzyl-2,7-

(formule I : R = $CH_3$ et R' = $CH_2$- 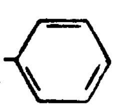 )

On a dissous 1 g (0,0049 mole) de DAP dans 20 ml de chloroforme. On a ensuite ajouté de l'iodure de méthyle en excès (3 ml ; 0,048 mole). Le mélange réactionnel obtenu a été agité à la température ambiante

pendant 24 heures. Un solide jaune a alors précipité. Ce solide jaune a été séparé du milieu réactionnel par filtration, lavé avec du chloroforme et séché sous vide. On a obtenu 1,61 g (0,0047 mole) d'iodure de N-méthyl-2,7-diazapyrène ($MDAP^+I^-$) (rendement : 96 %).

Ce produit brut étant partiellement protoné a été soumis à une déprotonation par traitement avec de l'eau légèrement alcaline (pH ≃ 7,8 ; eau + traces de $NH_4OH$).

Le produit brut a ensuite été recristallisé dans du méthanol bouillant et on a obtenu des cristaux jaunes.

Ce produit présentait les caractéristiques physicochimiques ci-après :

| Analyse élémentaire : $C_{15}H_{11}N_2I$ ; $0,25H_2O$ M = 350,7 | | | |
|---|---|---|---|
| Calculé<br>Trouvé | C 51,37 ;<br>C 51,31 ; | H 3,31 ;<br>H 3,19 ; | N 7,99<br>N 8,05 |

Spectre RMN $^1H$ (solvant $D_2O$ / $CF_3COOD$ ; référence TMPS)
10.05 ppm (s)
10,03 ppm (s)
$\delta$ o = 8,89 ppm (J = 9,19 Hz A3, q)
5,00 ppm (s) méthyle

Le produit obtenu ci-dessus (1 g ; 0,0029 mole) a ensuite été mis en suspension dans 150ml d'acétonitrile anhydre et on y a ajouté du bromure de benzyle (3ml ; 0,025 mole), préalablement filtré sur une colonne d'$Al_2-O_3$ neutre. Le mélange réactionnel résultant a été chauffé au reflux sous agitation pendant 12 heures.

Le produit jaune obtenu a été filtré, lavé avec du chloroforme et séché sous vide.

Rendement : 1,15 g (0,0022 mole) soit 77 %.

Le produit brut ainsi obtenu a été agité à l'obscurité pendant 24 heures avec de l'eau (100 ml) et du chlorure d'argent (obtenu à partir de 3 g de $AgNO_3$ et de NaCl, correctement lavé avec de l'eau). Le solide formé a été filtré, lavé avec de l'eau chaude, puis le filtrat a été concentré sous vide et dilué avec de l'acétone (90 ml) et un précipité jaune s'est formé. On a laissé ce mélange pendant une nuit et on l'a filtré et séché sous vide.

Rendement : 0,81 g (0,00213 mole) 73 %.

Le produit obtenu est le dichlorure de N-méthyl-N'-benzyl-2,7-diazapyrènequi présente les caractéristiques physico-chimiques ci-après :

| Analyse $C_{22}H_{18}N_2Cl_2$ M = 381,3 | | | |
|---|---|---|---|
| Calculé<br>Trouvé | C 69,29 ;<br>C 69,07 ; | H 4,72 ;<br>H 4,89 ; | N 7,35<br>N 7,38 |

Spectre RMN $^1H$ (solvant : $D_2O$ ; Référence : TMPS)
9,98 ppm (s)
9,88 ppm (s)
8,675 ppm (AB - q)
7,41 ppm (m - benzyle)
6,24 ppm (s)
4,81 ppm (s , méthyle)

EXEMPLE 2 : Préparation du dérivé diazapyrénique bis-$MDAP^{4+}$

(formule II : R = R' = $CH_3$

Ⓩ = $-CH_2-C_6H_4-CH_2-C_6H_4-CH_2-$

On a fait réagir le 4,4-bis(bromométhyl)diphénylméthane (0,174 g) avec le 2,7-diazapyrène (0,2 g) dans de l'acétonitrile anhydre (15 ml) au reflux et sous agitation pendant 4 heures. Le précipité jaune qui s'est formé a été séparé par filtration, lavé avec du chloroforme et séché sous vide.

Rendement : 0,34 g ; 85 %

Le produit brut a été recristallisé dans de l'eau chaude et analysé. Il s'agit du produit de formule :

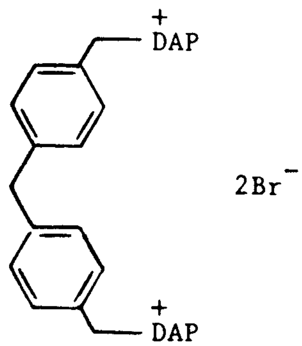

qui présente les caractéristiques physico-chimiques ci-après :

| Analyse élémentaire $C_{43}H_{30}N_4Br_2$, $3H_2O$ M = 816,6 | | | |
|---|---|---|---|
| Calculé<br>Trouvé | C 63,24<br>C 63,17 | H 4,44<br>H 4,25 | N 6,86<br>N 6,65 |

Spectre RMN $^1H$ (solvant : $D_2O$ / $CF_3COOD$ ; référence TMPS)
10,09 ppm (s)
10,00 ppm (s)
δo - 8,81 ppm (J = 9,37 Hz AB-q)
δo = 7,91 ppm (J = 7,61 Hz AB-q)
6,29 ppm (s)
3,98 ppm (s)

Ce produit a été mis en suspension(0,3 g) dans 50 ml d'acétonitrile et 0,5 ml d'iodure de méthyle, et chauffé au reflux pendant 4 heures sous agitation. On a obtenu un solide rouge-brun insoluble dans l'acétonitrile. Ce solide a été filtré, lavé avec du chloroforme et séché sous vide.
Rendement : 0,37g ; 90 %.

On a procédé ensuite à un échange d'ion en mettant en suspension le disel ainsi obtenu (iodure/bromure) avec du chlorure d'argent dans de l'eau et en agitant à la température ambiante pendant 24 heures. Le solide formé a été séparé par filtration et lavé avec de l'eau chaude. Le produit brut (solide jaune, très soluble dans l'eau) a été recristallisé plusieurs fois dans du méthanol ou un mélange méthanol/éther.

Selon un autre mode opératoire, on peut effectuer l'étape de méthylation en mélangeant le produit de formule ci-dessus avec du bromure de méthyle à 40-50 °C dans des flacons fermés pendant deux à trois jours. On obtient alors un produit jaune soluble dans l'eau.

Le produit obtenu dans l'un ou l'autre des cas présentait les caractéristiques physiocochimiques ci-après :

| Analyse élémentaire : $C_{45}H_{36}N_4Cl_4$ , $9H_2O$ M = 937 | | | |
|---|---|---|---|
| Calculé<br>Trouvé | C 57,70<br>C 57,80 | H 5,81<br>H 5,04 | N 5,98<br>N 5,86 |

Spectre RMN $^1H$ (solvant : $D_2O$ ; référence TMPS).
10,15 ppm (s)
10,09 ppm (s)
8,87 ppm (AB-q ; J = 9,47 Hz)
7,55 ppm (AB-q ; J = 8,18 Hz)
6,39 ppm (s)
5,20 ppm (s) méthyle
4,13 ppm (s)

EXEMPLE 3 : Préparation des dérivés bis-$DAP^{4+}$

(formule II R = R' = $CH_3$ ;

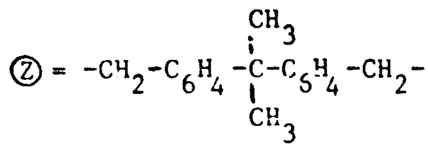

ou $-CH_2-C_6H_4-O-C_6H_4-CH_2-$

On a procédé selon le mode opératoire décrit à l'exemple 2 en remplaçant le 4,4'-bis(bromométhyl)-diphénylméthane respectivmeent par :

- le 4,4'-bis(bromométhyl)diphényl-2,2'-propane.
- le 4,4'-bis(bromométhyl) diphényléther et on a obtenu respectivement :
- le composé de formule :

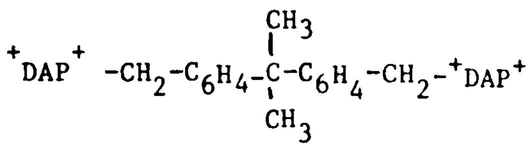

dont le spectre RMN est le suivant :
10,28 ppm (s) ; 10,21 ppm (s) ; 9,00 ppm (q-AB) 7,7 ppm (q-AB) ; 6,52 ppm(s) ; 5,14 ppm (s) ; 2,39 ppm(s)

- et le composé de formule : $^+DAP^+-CH_2-C_6H_4-O-C_6H_4-CH_2-{}^+DAP^+$ dont le spectre RMN est le suivant 10,27 ppm(s) ; 10,18 ppm(s) ; 8,97 ppm (q-AB) ; 7;82 ppm(d) 7,34 ppm(d) ; 6,51 ppm(s) ; 5,10 ppm (s).

EXEMPLE 4 : Préparation du tosylate de 2,4-dinitrophényl-2,7-diazapyrénium (intermédiaire de synthèse)

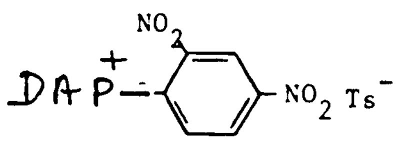
dénommé ci-après $DAP^+$-DNP $Ts^-$

0,81 g (2,4 $10^{-3}$ mole) de tosylate de 2,4 dinitrophényle (préparé par réaction du chlorure de tosyle et du 2,4-dinitrophényl) et 0,49 g ($4.10^{-3}$ mole) de 2,7-diazapyrène ont été chauffés à reflux dans un mélange (50/50 V/V) de chloroforme et d'acétonitrile.

Le précipité jaune formé a été filtré et redissous dans du méthanol ; Le nouveau précipité formé a été lavé dans un excès d'éther. Le précipité jaune obtenu a ensuite été séché sous vide.

Rendement : 0,96 g 73 %
Point de fusion : 252-254 ° C (décomposition)

| L'analyse élémentaire de ce produit était la suivante : | | | |
|---|---|---|---|
| Calculé<br>Trouvé | C 59,78<br>C 59,82 | H 3,34<br>H 3,10 | N 10,33<br>N 10,41 |

Spectre RMN $^1H$ (solvant $D_2O$ ; référence TMPS)
10,285 ppm (s)
9,975 ppm (s)
9,4 ppm (s)
9,0 ppm (d,d J = 0,05 p)
8,90 ppm (d J = 0,05 p)

8,675 ppm (d J = 0,05 p)
8,5 ppm (d J = 0,05 p)
7,575 ppm (d J = 0,041 p)

EXEMPLE 5 : Préparation du dérivé diazapyrénique de formule I

avec R = H et R' = $-(CH_2)_2-NH_2$

On a opéré selon le mode opératoire du procédé C en utilisant l'amine de formule $H_2N-(CH_2)_2NH_2$ dont une fonction amine a été protégée par le groupe carbobenzoxy $C_6H_4-CH_2-O-CO-$On a dissous 0,44 g de $DAP^+$-DNP $Ts^-$ dans 40 ml de méthanol anhydre et on a mis la solution obtenue dans une ampoule à brome.

D'autre part, on a préparé l'amine à partir du sel d'ammonium correspondant et on l'a dissoute (1,6 . $10^{-3}$ mole) dans 20 ml de méthanol anhydre sous agitation. On a ensuite ajouté quelques gouttes de collidine au mélange réactionnel toujours sous agitation. Puis, on a ajouté goutte à goutte pendant une heure la solution de $DAP^+$-DNP $Ts^-$. La solution est passée du rouge profond au brun. L'agitation a été poursuivie pendant encore une heure après quoi le solvant a été éliminé sur büchner. Ensuite, on a ajouté 300ml d'éther anhydre et il s'est formé un précipité légèrement brun. La solution a été agitée pendant encore 1 h 30 minutes et filtrée. Le solide obtenu a été séché pendant une nuit dans un dessicateur.
Rendement : 0,41 g ; 94 %.

Le composé obtenu a les caractéristiques physicochimiques ci-après :

| Analyse élémentaire M = 537 | | | | | | |
|---|---|---|---|---|---|---|
| Calculé | C | 64,53 | H : | 5,94 | N: | 7,51 |
| Trouvé | C | 64,58 | H | 5,00 | N: | 7,32. |

RMN(ppm) 9,87(s) ; 8,53(d) ; 8,77(d) ; 9,92(s)
5,19(t) ; 3,98(t) ; 6,6 (s) ; 4,8(s)
7,1 (s) ; 7,2(d) ; 7,7 (d) ; 2,07(s)

On a ensuite déprotégé la fonction amine en faisant réagir le produit obtenu avec un mélange d'acide acétique et d'acide bromhydrique à la température ambiante.

Le produit obtenu répondait à la formule $^+DAP^+-(CH_2)_2-NH_3^+$ , $3Br^-$dont l'analyse élémentaire est la suivante :

| | | | | | | |
|---|---|---|---|---|---|---|
| Calculé<br>Trouvé | C | 39,19<br>34,11 | H: | 3,28<br>3,22 | N | 8,57<br>7,43 |

Le spectre RMN ($D_2O$, TMPS) de ce produit a revélé des pics à 9,93ppm, 8,80 ppm, 8,86 ppm , 10,09 ppm, 5,56 ppm et 4,00 ppm. Par action d'une base sur le produit obtenu on obtient le produit attendu.

EXEMPLE 6 : Préparation du dibromure de N-méthyl-N'-hydroxypentyl-2,7-diazapyrénium

(formule I : R = $CH_3$ ; R = $-(CH_2)_5-OH$ )

Du 5-amino-1-pentanol a été dissous dans du méthanol anhydre et mis dans un récipient sec de 100 ml sous argon et dans des conditions anhydres.

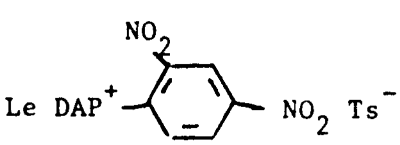

a été dissous dans du méthanol anhydre et versé goutte à goutte par l'intermédiaire d'une ampoule à brome dans la solution de 5-amino-1-pentanol sous agitation à 20 ° C. La solution est obtenue rouge, puis rouge foncé. L'agitation a été poursuivie encore pendant deux heures et le méthanol a été ensuite éliminé sous vide presque jusqu'à siccité. On a alors ajouté au résidu 250 ml d'éther et un précipité brun s'est formé ; il a été séparé par filtration , repris par du méthanol et séché.

Par chromatographie couche mince sur gel de silice éluée par un mélange $CHCl_3/CH_3OH$ (5% - 95%) on a noté une seule tache. Rendement 72 % - Point de fusion supérieur à 250 ° C RMN $D_2O$/TMPS.

δ = 9,65 s
δ = 9,3 s
δ = 8,23 q
δ = 7,49 d ) Systeme AB
δ = 7,18 d )
δ = 5,11 t
δ = 3,67 t
δ = 2,32 p
δ = 1,60 pp

<u>EXEMPLE 7</u> : <u>Préparation du dérivé diazapyrénique de formule I</u>

avec

R = R' =

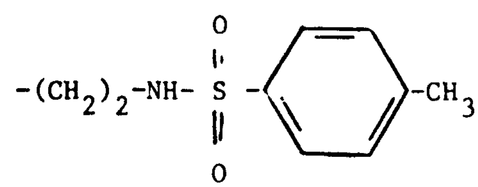

On a dissous 25 mg ($1,22.10^{-4}$ mole) de DAP dans 5 ml de collidine froide. On a ajouté à cette solution immédiatement l'un après l'autre :

48,2 mg ($2,44.10^{-4}$ mole) de tosyl-aziridine et
38,4 mg ($2,44.10^{-4}$ mole) de chlorure de collidinium.

L'ensemble a été chauffé de 20 ° C à 100 ° C sous agitation pendant 2 heures. La solution est passée du brun foncé au jaune foncé et a été décantée , puis évaporée jusqu'à siccité par chauffage à 50 ° C.

Le produit obtenu a donné une seule tache en chromatographie couche mince sur gel de silice (élution méthanol/$CH_2Cl_2$ 2%/98%).

Rendement : 48 %.

Le spectre RMN du produit est le suivant :

λ = 9,5 s
δ = 8,4 s
δ = 7,8 )
δ = 7,75 ) système AB
δ = 7,43 )
δ = 7,39 )
δ = 3,5 t
δ = 3,2 t
δ = 2,4 s

<u>EXEMPLE 8</u> : <u>Préparation du dérivé diazapyrénique de formule I</u>

avec R = R' = $R_1Y^-$

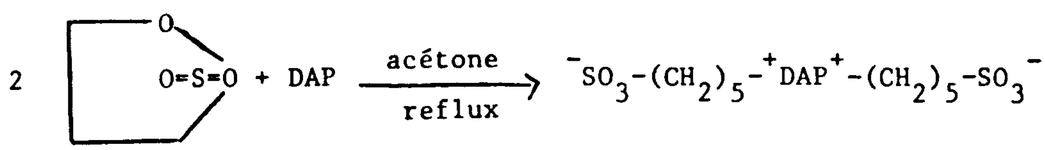


On a dissous partiellement 25 mg de DAP dans de l'acétone anhydre (1ml) et on a chauffé au reflux ; le propanesultone a été ajouté (45 mg) et le mélange a été agité au reflux pendant une nuit.

Le produit obtenu a donné une seule tache en chromatographie couche mince sur gel de silice (éluant: méthanol/$CH_3Cl_3$ 10%/90%). L'acétone a été éliminée sous pression réduite.

Le spectre RMN dans $CF_3\ CO_2^-\ D^+$ a donné les pics suivants :

$\delta$ = 8,26 s
$\delta$ = 6,9 s
$\delta$ = 3,44 t
$\delta$ = 1,32 t
$\delta$ = 0,77 t

EXEMPLE 9 : Préparation du dérivé diazapyrénique de formule I avec :

$$R=CH_3\ ;\ R' = -(CH_2)_3-\overset{CH_3}{\underset{CH_3}{N^+}}-(CH_2)_3-\overset{CH_3}{\underset{CH_3}{N^+}}-C_{18}H_{37}$$

Ce dérivé est préparé selon le procédé C défini précédemment.

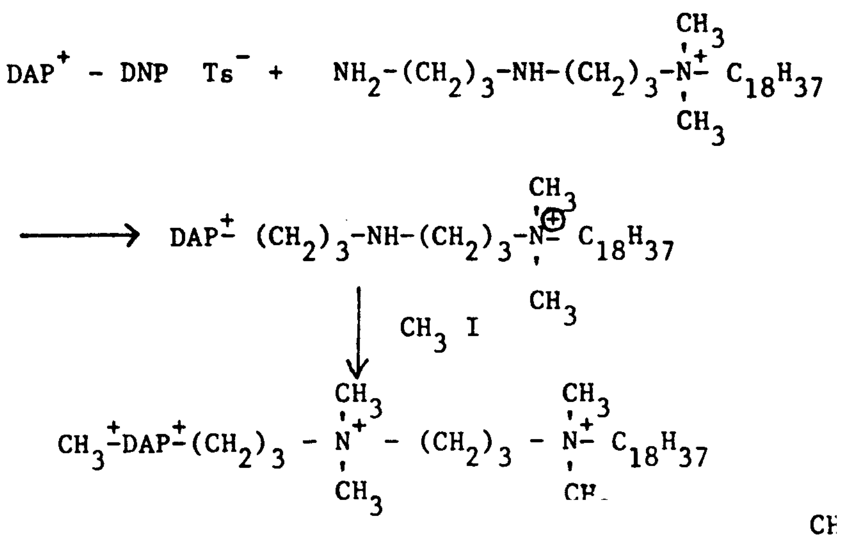


A - préparation du composé

$$NH_2-(CH_2)_3-NH-(CH_2)_3-\overset{CH_3}{\underset{CH_3}{N^+}}-C_{18}H_{37}$$

a) $CH_3NH + C_{18}H_{37}\ Br \rightarrow C_{18}H_{37}\ N(CH_3)_2$

On a fait réagir au reflux 1,6 g du bromure $C_{18}H_{37}\ Br$ en solution dans 10 ml d'éthanol avec 10 ml d'une solution à 33% de l'amine $(CH_3)_2\ NH$ dans l'éthanol et on a obtenu 1,42 g de l'amine tertiaire $C_{18}H_{37}\ N(CH_3)_2$.

Cette amine a été ensuite purifiée, après avoir réduit à siccité le mélange réactionnel obtenu, par extraction avec du $CHCl_3$ et de l'eau basique (contenant du LiOH). La fraction $CHCl_3$ a été filtrée et séchée.

$$b)\ C_{18}H_{37}N(CH_3)_2 + Br-(CH_2)_3-Br \longrightarrow C_{18}H_{37}-\overset{CH_3}{\underset{CH_3}{N^{+}}}-(CH_2)_3Br\ Br^-$$

On a fait ensuite réagir 1,4 g de l'amine ainsi obtenue avec 4 ml du dibromure Br - $(CH_2)_3$ - Br et 10 ml d'éthanol en portant le mélange réactionnel au reflux pendant 20 heures. Les solvants ont ensuite été éliminés et le résidu a été dissous dans 5 ml de $CHCl_3$. La solution obtenue a été versée dans de l'éther jusqu'à précipitation. Le précipité rose formé a été filtré et séché. On a obtenu 1,8 g de produit qui a été recristallisé dans 40 ml d'acétate d'éthyle. On a obtenu 1,5 g de cristaux (rendement 98%) du bromure attendu dont le spectre RMN $^1H$ est le suivant :
δ(ppm) : 0,875(t) ; 1,25(s) ;
1,45(t) ; 2,39(p) ; 3,00(p) ;
3,39(p) ; 3,45(t) ; 3,58(t) ;
3,80(t).

c) 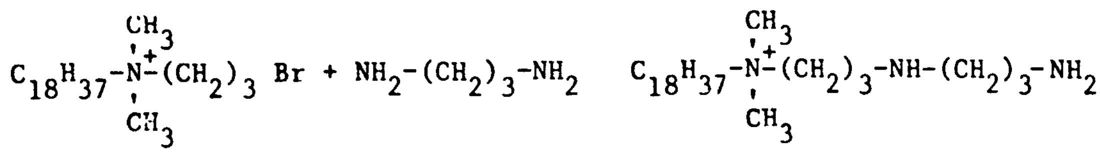

On a fait réagir 499 mg du bromure obtenu avec 83,4 µl de propylène diamine en chauffant à 110 °C pendant 4h 30 min. Le solvant a été éliminé et le résidu isolé selon les méthodes classiques. Le spectre RMN $^1H$ de l'amine obtenue est le suivant :
δ(ppm) : 0,78(t) ; 1,15(s) ; 1,25(m) ; 1,6(t) ;
1,61(t) ; 1,8(m) ; 2,55(t) ; 2,72(t) ;
3,00(s) ; 3,15(t) ; 3,40(m) ;

B. Préparation du composé de l'invention

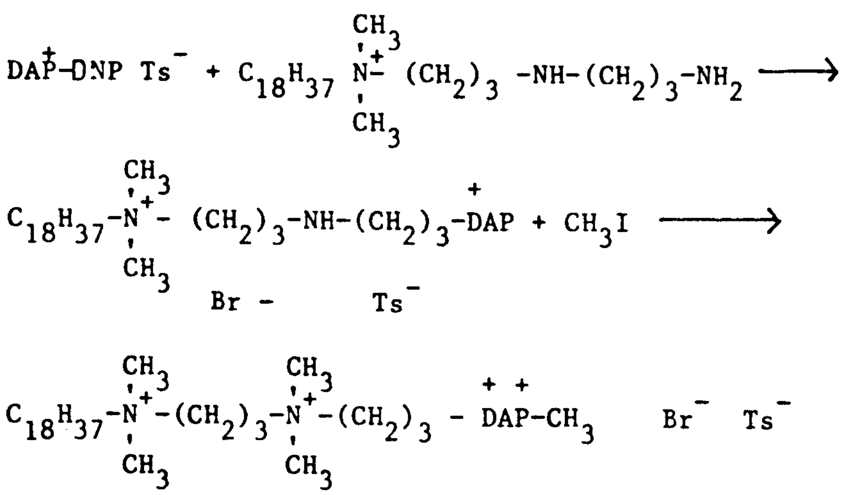

On a opéré selon le mode opératoire du procédé C en dissolvant 43,5 mg (8,05 $10^{-5}$ mole) de DAP - DNP $Ts^-$ dans 15 ml de méthanol et en ajoutant goutte à goutte la solution obtenue à une solution de l'amine (39,5 mg ; 8,05 $10^{-5}$ mole) dans 6 ml de méthanol. L'addition lente a duré 1/2 heure et a été suivie d'une agitation pendant 4h 30 min sous azote après quoi le solvant a été éliminé. Ensuite on a ajouté de l'éther et il s'est formé un précipité, lequel a été isolé et purifié selon les méthodes classiques.

On a fait ensuite réagir 18,6 mg (2,2 $10^{-5}$mole du produit ainsi obtenu avec de l'iodure de méthyle (3 µl ; 2,2 $10^{-4}$ mole) dans le DMF à 70 °C pendant 15 heures. Le produit obtenu a été passé sur une colonne échangeuse d'ions ($Cl^-$) et on a obtenu le composé de formule ci-après :

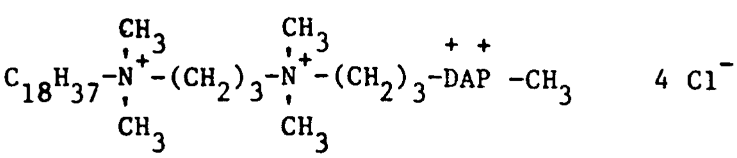

dont le poids moléculaire est de 787 et le spectre RMN présente les caractéristiques ci-après :

| | | |
|---|---|---|
| δ = 0,78 t | δ = 3,00 s | δ = 10,44 s |
| δ = 1,15 s | δ = 3,24 s | δ = 10,52 s |
| δ = 1,25 m | δ = 3,40 m | |
| δ = 1,6 t | δ = 5,05 s | |
| δ = 1,8 m | δ = 5,45 t | |
| δ = 2,55 t | δ = 9,09 système AB | |
| δ = 2,72 t | δ = 10,26 s | |

EXEMPLE 10 : Préparation du dérivé diazapyrénique de formule I avec

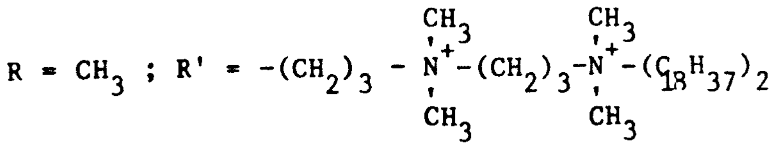

Ce dérivé est également préparé selon le procédé B défini ci-dessus en faisant réagir le DAP - DNP Ts⁻ sur l'amine de formule

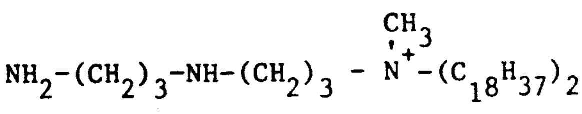

A. Préparation de l'amine

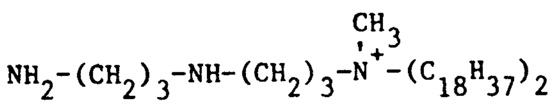

a)

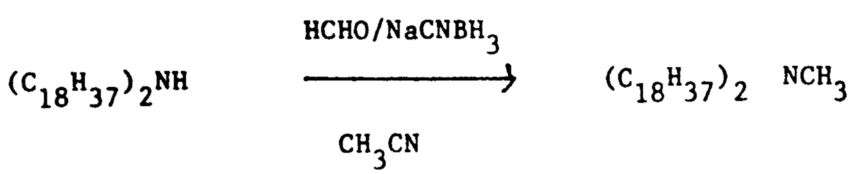


On chauffe à 80°C 1,5 g de l'amine dans 20 ml de $CH_3CN$ ; on ajoute 1,1 ml de formaldéhyde et on refroidit la solution. Ensuite on ajoute 0,19 g de $NaCNBH_3$, puis 1 ml d'acide acétique glacial est ajouté lentement pendant 10 minutes. La solution est alors agitée pendant deux heures, puis on ajoute à nouveau 2 ml d'acide acétique glacial pendant 1/2 heure. La solution obtenue est versée dans de l'éther, extraite 3 fois avec 20 ml de NaOH 1M et 1 fois avec 20 ml de saumure. La phase éthérée a été séchée sur $K_2CO_3$, filtrée et le solvant a été éliminé. On a obtenu 1,39 g du produit attendu.

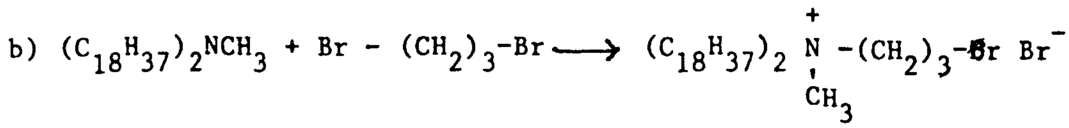

On a agité au reflux pendant 14 jours :

200 mg de l'amine tertiaire (obtenue au point a) dans 3 ml de $CH_2Cl_2$, 1 ml du dibromure $Br\text{-}(CH_2)_3\text{-}Br$ et un cristal de Ki. Le solvant a été ensuite éliminé et on a obtenu 274 mg du produit attendu (rendement 100%)

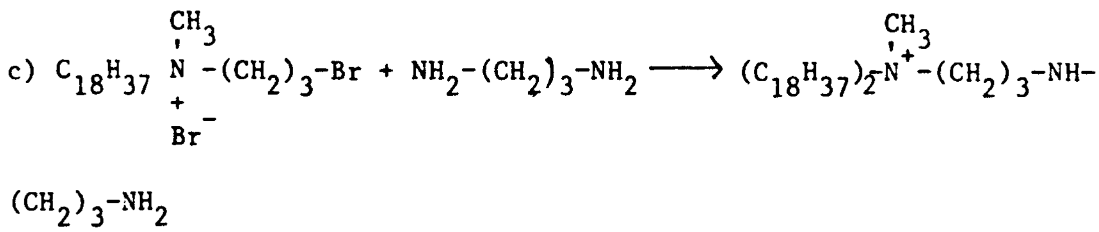

On a opéré selon le mode opératoire décrit à l'exemple 9.c et on a obtenu le produit caractérisé par le spectre RMN $^1H$ ci-après :
δ(ppm) : 0,83(t) ; 1,20(s) ; 1,67(t) ; 1,92(p) ;
2,69(t) ; 2,85(t) ; 3,21(t) ; 3,31(t) et
3,57(t).

B. <u>Préparation du composé de l'invention</u>

On a répété le mode opératoire de l'exemple 9 B et on a obtenu successivement :

1) le composé de formule :

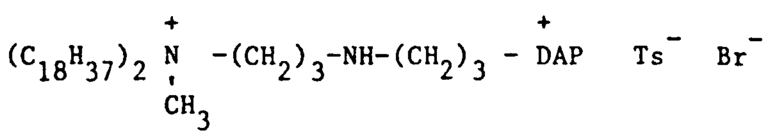

dont le spectre RMN $^1H$ est le suivant :
δ (ppm): 0,94(t) ; 1,33(s) ; 1,59(p) ; 2,29(s);
2,78(p) ; 3,11(s) ; 3,20(t) ; 5,35(t);
7,14(d) ; et 7,61(d) (système AB)
8,63 d et 8,76 d (système AB) ; 9,84(s) et 10,11(s)

2) le composé de formule :

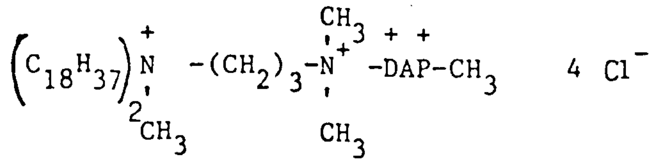

caractérisé par le spectre RMN $^1H$ (DMSO d 6) ci-après :
δ(ppm) : 4,32(s) ; 4,67(t) ; 6,5(d) et 6,83(d) système AB) ;
8,34(q) et 9,73(s) système AB) ; 9,94(s)

**Revendications**

**Revendications pour les Etats contractants suivants : AT, BE, CH, DE, FR, GB, GR, IT, LI, LU, NL, SE**

**1.** Composé diazapyrénique de formule :

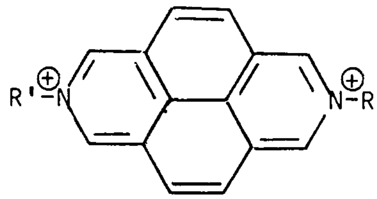

$2X^-$ (I)

dans laquelle R' est l'hydrogène ou un groupe alkyle en $C_1$-$C_6$ et R est l'hydrogène ou un groupe alkyle substitué choisi parmi les groupes de formules $-(CH_2)_n$-SH, $-(CH_2)_n$-OH et $-(CH_2)_n$-$NH_2$, dans lesquels n est compris entre 1 et 10, $X^-$ est un anion, avec la condition que R et R' ne soient pas en même temps l'hydrogène.

**2.** Dichlorure de N-méthyl-N'-benzyl-2,7-diazapyrénium.

**3.** Composé diazapyrénique de formule :

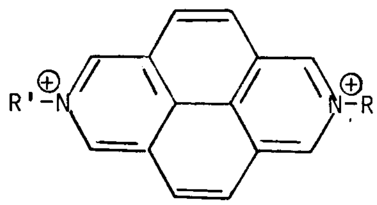

$2X^-$ (I)

dans laquelle les deux groupes R et R' sont identiques et représentent (a) le groupe de formule $-R_1Y$-, dans laquelle $R_1$ est $-(CH_2)_3$- et $Y^-$ l'ion sulfonate, $SO_3^-$ , ou (b) le groupe de formule :

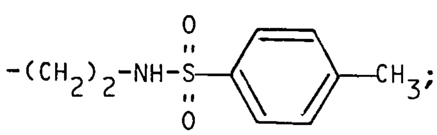

; et

$X^-$ est un anion.

**4.** Composé diazapyrénique de formule :

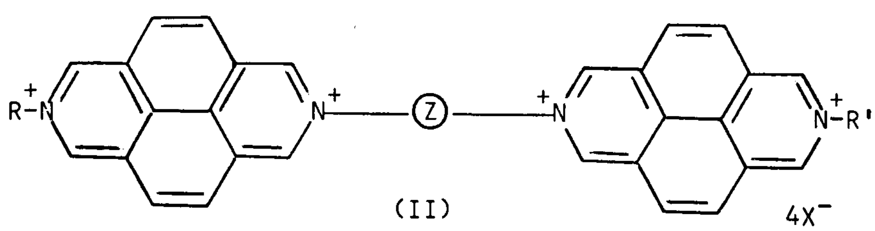

(II) $4X^-$

dans laquelle R et R' sont identiques ou différents et représentent chacun l'hydrogène ou un groupe alkyle en $C_1$-$C_6$; $X^-$ est un anion et Z est choisi parmi les radicaux divalents de formules :

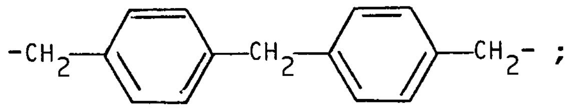

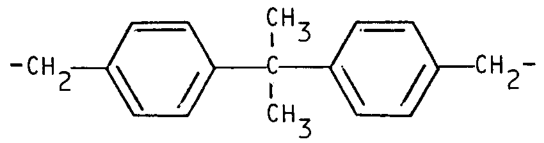

; et

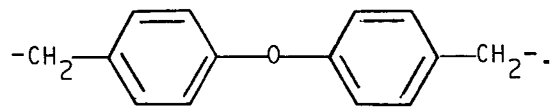

5. Composé diazapyrénique de formule :

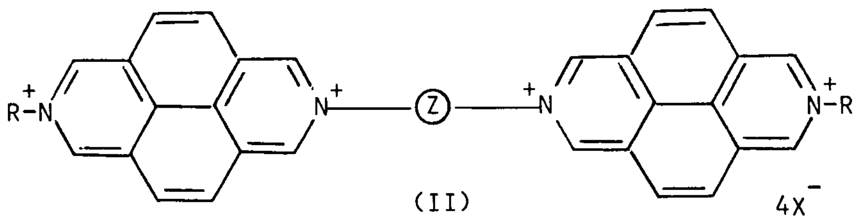

(II)

dans laquelle R et R' sont des groupes méthyle, $X^-$ est l'ion chlorure, et Z est $-CH_2-C_6H_4-CH_2-C_6H_4-CH_2-$.

6. Procédé de photoclivage des acides nucléiques, caractérisé en ce qu'il consiste à faire réagir, en présence ou en l'absence d'air (oxygène) ou d'EDTA, une solution aqueuse d'un acide nucléique avec une solution d'un dérivé diazapyrénique tel que défini dans l'une quelconque des revendications 1 à 5 et à soumettre le mélange réactionnel obtenu à une irradiation à la lumière visible.

7. Procédé pour l'obtention d'un composé diazapyrénique selon la revendication 1, caractérisé en ce qu'il comporte essentiellement deux étapes de N-substitution du 2,7-diazapyrène ou DAP selon les schémas réactionnels ci-après :

$DAP + RX_1 \rightarrow DAP^{+}\text{-}RX_1^{-}$
$DAP^{+}\text{-}RX_1^{-} + R'X_2 \rightarrow R^{+}\text{-}DAP^{+}\text{-}R'X_1X_2$

dans lesquels $X_1$ et $X_2$, identiques ou différents, ont la même signification que X; et en ce qu'il consiste éventuellement à transformer, par échange d'ions, le composé obtenu en le composé correspondant, dans lequel $X_1$ et $X_2$ sont identiques.

8. Procédé pour l'obtention d'un composé diazapyrénique de formule I selon la revendication 1, dans laquelle R est un groupe alkyle substitué par un groupe hydroxy et R' est un groupe alkyle, caractérisé en ce qu'il consiste à fabriquer d'abord un sel de 2-dinitrophényl-2,7-diazapyrénium par réaction du 2,7-diazapyrène avec un sel de 2,4-dinitrophényle, tel que le tosylate, dans un solvant approprié, puis à faire réagir ledit intermédiaire avec une amine substituée par un radical R et, ensuite, à effectuer une N-substitution avec un halogénure de formule R'X selon la revendication 7.

**9.** Procédé pour l'obtention d'un composé diazapyrénique de formule I selon la revendication 1, dans laquelle R est un groupe alkyle substitué par un groupe amino et R' est un groupe alkyle, caractérisé en ce qu'il consiste à fabriquer d'abord un sel de 2-dinitrophényl-2,7-diazapyrénium par réaction du 2,7-diazapyrène avec un sel de 2,4-dinitrophényle, tel que le tosylate, dans un solvant approprié, puis à faire réagir ledit intermédiaire avec une diamine dont l'une des fonctions amine est protégée, laquelle est ensuite déprotégée avant l'étape de N-substitution avec un halogénure de formule R'X selon la revendication 7.

**10.** Procédé pour l'obtention d'un composé diazapyrénique de formule I selon la revendication 3, dans laquelle R et R' sont identiques et représentent un radical $R_1Y^-$ tel que défini dans la revendication 1, caractérisé en ce qu'il consiste à faire réagir ensemble du 2,7-diazapyrène (DAP) partiellement dissous dans de l'acétone anhydre et du propanesultone à la température de reflux.

**11.** Procédé pour l'obtention d'un dérivé diazapyrénique de formule I selon la revendication 3, dans laquelle R et R' sont identiques et représentent le groupe de formule :

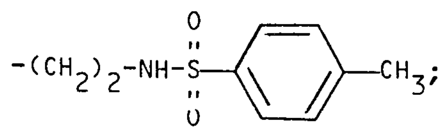

caractérisé en ce qu'il consiste à faire réagir du 2,7-diazapyrène avec l'azidine en présence de collidine et de chlorure de collidinium.

**12.** Procédé pour l'obtention d'un dérivé diazapyrénique selon la revendication 4, caractérisé en ce qu'il consiste à faire réagir deux molécules de 2,7-diazapyrène (DAP) avec une molécule d'un dihalogénure de formule $X_1$-Z-$X_2$, dans laquelle Z est tel que défini dans la revendication 4, $X_1$ et $X_2$, identiques ou différents, ont la même signification que X, et à procéder ensuite à la N-substitution des azotes restant dans les molécules de DAP selon le schéma réactionnel ci-après :

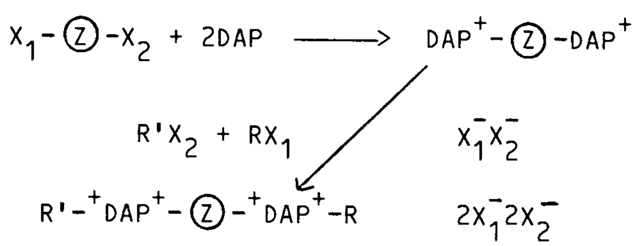

**Revendications pour l'Etat contractant suivant : ES**

**1.** Procédé pour l'obtention de dérivés diazapyréniques de formule

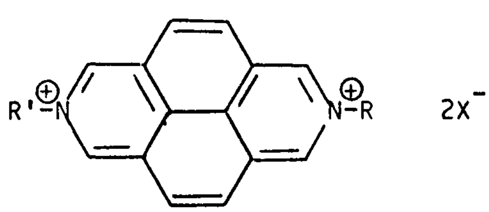

(I)

dans laquelle R' est l'hydrogène ou un groupe alkyle en $C_1$-$C_6$ et R est l'hydrogène ou un groupe alkyle substitué choisi parmi les groupes de formules $-(CH_2)_n$-SH, $-(CH_2)_n$-OH et $-(CH_2)_n$-$NH_2$, dans lesquels n est compris entre 1 et 10, $X^-$ est un anion, avec la condition que R et R' ne soient pas en même temps l'hydrogène, caractérisé en ce qu'il comporte essentiellement deux étapes de N-substitution du 2,7-diazapyrène ou DAP selon les schémas réactionnels ci-après :

$DAP + RX_1 \rightarrow DAP^+\text{-}RX_1^-$
$DAP^+\text{-}RX_1^- + R'X_2 \rightarrow R^+\text{-}DAP^+\text{-}R'X_1X_2$

dans lesquels $X_1$ et $X_2$, identiques ou différents, ont la même signification que X; et en ce qu'il consiste éventuellement à transformer, par échange d'ions, le composé obtenu en le composé correspondant, dans lequel $X_1$ et $X_2$ sont identiques.

**2.** Procédé pour l'obtention de dérivés diazapyréniques de formule I selon la revendication 1, dans laquelle R est un groupe alkyle substitué par un groupe hydroxy et R' est un groupe alkyle, caractérisé en ce qu'il consiste à fabriquer d'abord un sel de 2-dinitrophényl-2,7-diazapyrénium par réaction du 2,7-diazapyrène avec un sel de 2,4-dinitrophényle, tel que le tosylate, dans un solvant approprié, puis à faire réagir ledit intermédiaire avec une amine substituée par un radical R et, ensuite, à effectuer une N-substitution avec un halogénure de formule R'X selon la revendication 1.

**3.** Procédé pour l'obtention de dérivés diazapyréniques de formule I selon la revendication 1, dans laquelle R est un groupe alkyle substitué par un groupe amino et R' est un groupe alkyle, caractérisé en ce qu'il consiste à fabriquer d'abord un sel de 2-dinitrophényl-2,7-diazapyrénium par réaction du 2,7-diazapyrène avec un sel de 2,4-dinitrophényle, tel que le tosylate, dans un solvant approprié, puis à faire réagir ledit intermédiaire avec une diamine dont l'une des fonctions amine est protégée, laquelle est ensuite déprotégée avant l'étape de N-substitution avec un halogénure de formule R'X selon la revendication 1.

**4.** Procédé pour l'obtention d'un composé diazapirénique de formule

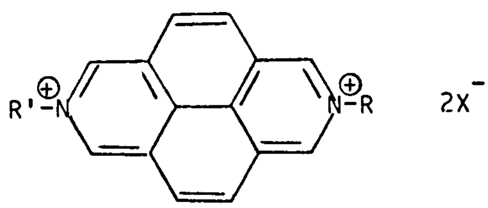 (I)

dans laquelle les deux groupes R et R' sont identiques et représentent le groupe de formule $-R_1Y-$, dans laquelle $R_1$ est $-(CH_2)_3-$, $Y^-$ l'ion sulfonate, $SO_3^-$ et $X^-$ est un anion, caractérisé en ce qu'il consiste à faire réagir ensemble du 2,7-diazapyrène (DAP) partiellement dissous dans de l'acétone anhydre et du propanesultone à la température de reflux.

**5.** Procédé pour l'obtention d'un composé diazapyrénique de formule

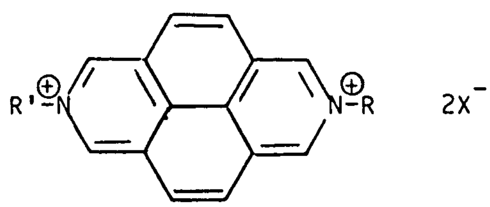 (I)

dans laquelle les deux groupes R et R' sont identiques et représentent le groupe de formule

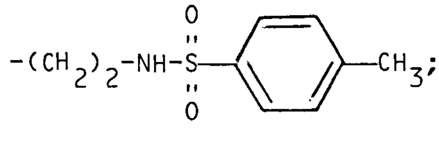 ; et

$X^-$ est un anion, caractérisé en ce qu'il consiste à faire réagir du 2,7-diazapyrène avec l'azidine en

présence de collidine et de chlorure de collidinium.

**6.** Procédé pour l'obtention d'un composé diazapyrénique de formule

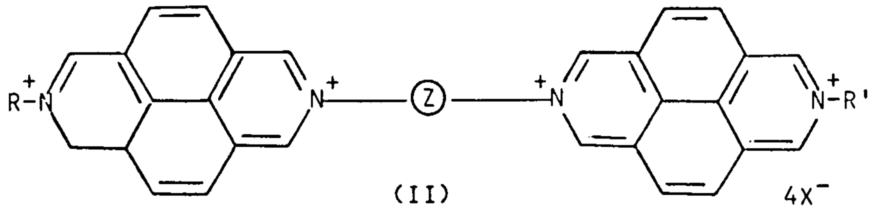

(II)

dans laquelle R et R' sont identiques ou différents et représentent chacun l'hydrogène ou un groupe alkyle en $C_1$-$C_6$ ; $X^-$ est un anion et Z est choisi parmi les radicaux divalents de formules :

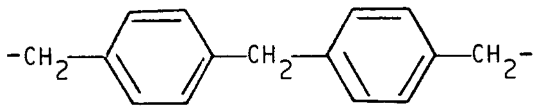 ;

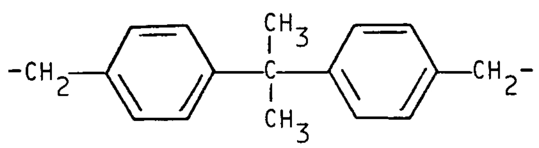 ; et

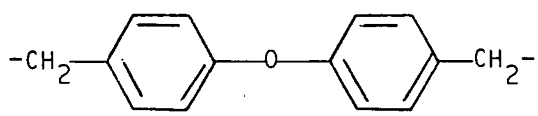

caractérisé en ce qu'il consiste à faire réagir deux molécules de 2,7-diazapyrène (DAP) avec une molécule d'un dihalogénure de formule $X_1$-Z-$X_2$, dans laquelle Z est tel que défini dans la revendication 4, $X_1$ et $X_2$, identiques ou différents, ont la même signification que X, et à procéder ensuite à la N-substitution des azotes restant dans les molécules de DAP selon le schéma réactionnel ci-après :

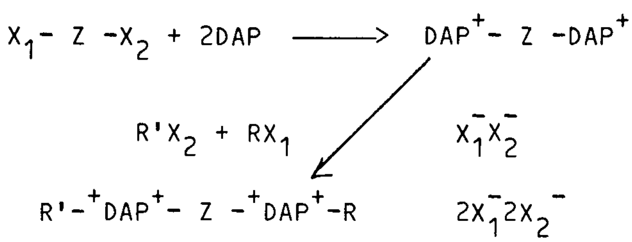

**7.** Procédé de photoclivage des acides nucléiques, caractérisé en ce qu'il consiste à faire réagir, en présence ou en l'absence d'air (oxygène) ou d'EDTA, une solution aqueuse d'un acide nucléique avec une solution d'un dérivé diazapyrénique tel qu'obtenu par les procédés selon l'une quelconque des revendications 1 à 6 et à soumettre le mélange réactionnel obtenu à une irradiation à la lumière visible.

**Claims**

**Claims for the following Contracting States : AT, BE, CH, DE, FR, GB, GR, IT, LI, LU, NL, SE**

**1.** A diazapyrene compound of formula :

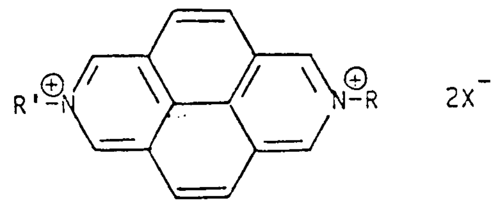

(I)

in which R' is hydrogen or an alkyl group with 1 to 6 carbon atoms and R is hydrogen or a substituted alkyl group selected among the groups of formulae $-(CH_2)_n-SH$, $-(CH_2)_n-OH$ and $-(CH_2)_n-NH_2$, in which n is between 1 and 10, $X^-$ is an anion at the condition that R and R' are not hydrogen at the same time.

**2.** N-methyl N'benzyl 2,7-diazapyrenium dichloride.

**3.** A diazapyrene compound of formula :

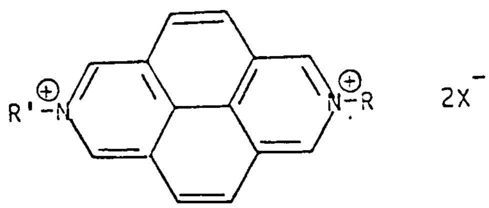

(I)

in which both groups R and R' are identical and represent (a) the group of formula $-R_1Y-$ in which $R_1$ is $-(CH_2)_3-$ and $Y^-$ is the sulfonato ion, $SO_3^-$, or (b) the group of formula :

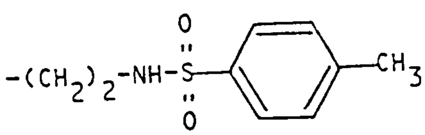

and

$X^-$ is an anion.

**4.** A diazapyrene compound of formula :

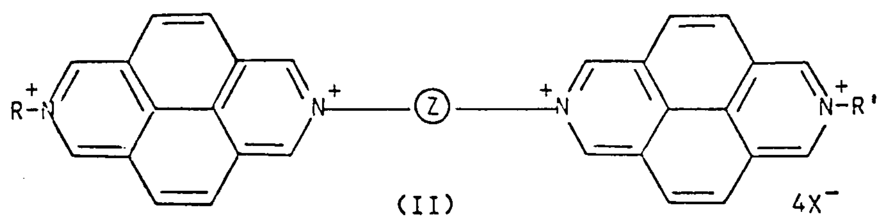

in which R and R' are identical or different and represent each hydrogen or an alkyl group with 1 to 6 carbon atoms; $X^-$ is an anion and Z is selected among the divalent radicals of formulae :

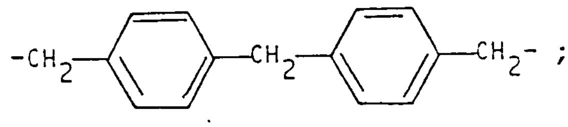

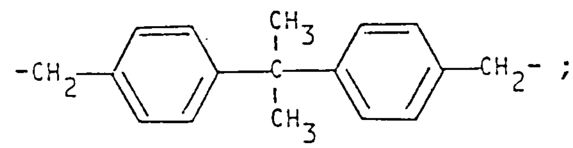

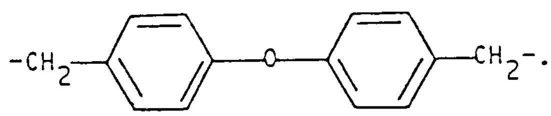

5. A diazapyrene compound of formula :

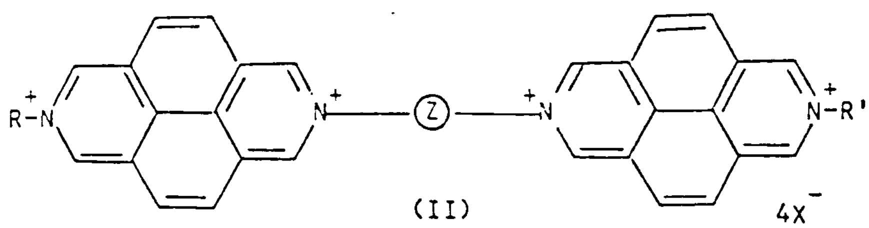

(II)

in which R and R' are methyl groups, $X^-$ is the chloride ion, and Z is $-CN_2-C_6H_4-CH_2-C_6H_4-CH_2-$.

6. Process for the photocleavage of nucleic acids, characterized in that it consists in reacting, in the presence or in the absence of air (oxygen) or EDTA, an aqueous solution of a nucleic acid with a solution of a diazapyrene derivative, as defined in any one of claims 1 to 5 and in subjecting the resulting reaction mixture to irradiation with visible light.

7. Process for preparing a diazapyrene compound according to claim 1, characterized in that it comprises essentially two steps of N-substitution of 2,7-diazapyrene or DAP according to the following reaction diagrams:

$DAP + RX_1 \rightarrow DAP^+\text{-}RX_1^-$
$DAP^+\text{-}RX_1^- + R'X_2 \rightarrow R^+\text{-}DAP^+\text{-}R'X_1X_2$

in which $X_1$ and $X_2$, identical or different, have the same meaning as X; and in that it consists, if appropriate, in converting, by ion exchange, the resulting compound into the corresponding compound, in which $X_1$ and $X_2$ are identical.

8. Process for preparing a diazapyrene compound of formula I according to claim 1, in which R is an alkyl group substituted by a hydroxy group and R' is an alkyl group, characterized in that it consists in producing first a 2-dinitrophenyl-2,7-diazapyrenium salt by reaction of 2,7-diazapyrene with a 2,4-dinitrophenyl salt, as tosylate, in an appropriate solvent, then in reacting said intermediate with an amine substituted by an R radical and, then, to carry out an N-substitution with a halogenide of formula R'X according to claim 7.

**9.** Process for preparing a diazapyrene compound of formula I according to claim 1, in which R is an alkyl group substituted by an amino group and R' is an alkyl group, characterized in that it consists in producing first a 2-dinitrophenyl-2,7-diazapyrenium salt by reaction of 2,7-diazapyrene with a 2,4-dinitrophenyl salt, as tosylate, in an appropriate solvent, then in reacting said intermediate with a diamine of which one of the amino groups is protected, which is then deprotected before the step of N-substitution with a halogenide of formula R'X according to claim 7.

**10.** Process for preparing a diazapyrene compound of formula I according to claim 3, in which R and R' are identical and represent a $R_1Y^-$ radical as defined in claim 1, characterized in that it consists in reacting together 2,7-diazapyrene (DAP), partly dissolved in anhydrous acetone, and propanesulton at the reflux temperature.

**11.** Process for preparing a diazapyrene derivative of formula I according to claim 3, in which R and R' are identical and represent the group of formula :

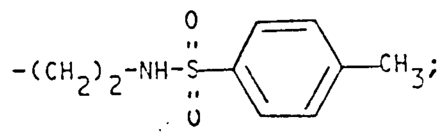

characterized in that it consists in reacting 2,7-diazapyrenium with azidine in the presence of collidine and collidinium chloride.

**12.** Process for preparing a diazapyrene derivative according to claim 4, characterized in that it consists in reacting two molecules of 2,7-diazapyrene (DAP) with a molecule of a dihalogenide of formula $X_1$-Z-$X_2$, in which Z is as defined in claim 4, $X_1$ and $X_2$, identical or different, have the same signification as X, and then in carrying out the N-substitution of the nitrogens remaining in the DAP molecules according to the following reaction diagram :

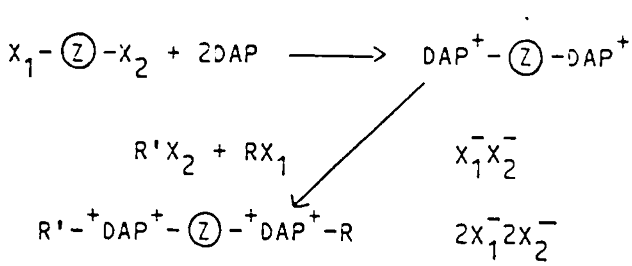

**Claims for the following Contracting State : ES**

**1.** A process for preparing diazapyrene derivatives of formula:

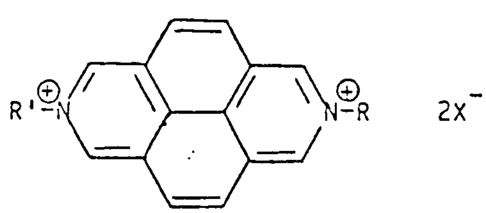

(I)

in which R' is hydrogen or an alkyl group with 1 to 6 carbon atoms and R is hydrogen or a substituted alkyl group selected among the groups of formulae $-(CH_2)_n-SH$, $-(CH_2)_n-OH$ and $-(CH_2)_n-NH_2$, in which n is between 1 and 10, $X^-$ is an anion at the condition that R and R' are not hydrogen at the same time, characterized in that it comprises essentially two steps of N-substitution of 2,7-diazapyrene or DAP according to the following reaction diagrams :

$DAP + RX_1 \rightarrow DAP^+\text{-}RX_1^-$
$DAP^+\text{-}RX_1^- + R'X_2 \rightarrow R^+\text{-}DAP^+\text{-}R'X_1X_2$

in which $X_1$ and $X_2$, identical or different, have the same meaning as X; and in that it consists, if appropriate, in converting, by ion exchange, the resulting compound into the corresponding compound, in which $X_1$ and $X_2$ are identical.

**2.** Process for preparing diazapyrene derivatives of formula I according to claim 1, in which R is an alkyl group substituted by a hydroxy group and R' is an alkyl group, characterized in that it consists in producing first a 2-dinitrophenyl-2,7-diazapyrenium salt by reaction of 2,7-diazapyrene with a 2,4-dinitrophenyl salt, as tosylate, in an appropriate solvent, then in reacting said intermediate with an amine substituted by an R radical and, then, in carrying out an N-substitution with a halogenide of formula R'X according to claim 1.

**3.** Process for preparing diazapyrene derivatives of formula I according to claim 1, in which R is an alkyl group substituted by an amino group and R' is an alkyl group, characterized in that it consists in producing first a 2-dinitrophenyl-2,7-diazapyrenium salt by reaction of 2,7-diazapyrene with a 2,4-dinitrophenyl salt, as tosylate, in an appropriate solvent, then in reacting said intermediate with a diamine of which one of the amino groups is protected, which is then deprotected before the step of N-substitution with a halogenide of formula R'X according to claim 1.

**4.** Process for preparing a diazapyrene compound of formula :

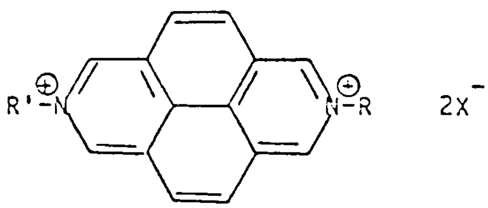

(I)

in which both groups R and R' are identical and represent the group of formula $-R_1Y-$, in which $R_1$ is $-(CH_2)_3-$ and $Y^-$ is the sulfonato ion, $SO_3^-$ and $X^-$ is an anion, characterized in that it consists in reacting together 2,7-diazapyrene (DAP), partly dissolved in an anhydrous acetone, and propansulton at the reflux temperature.

**5.** Process for preparing a diazapyrene compound of formula :

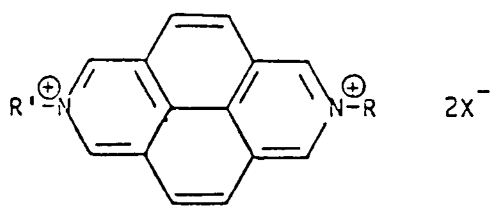

(I)

in which both groups R and R' are identical and represent the group of formula :

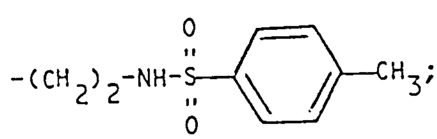

and

$X^-$ is an anion, characterized in that it consists in reacting 2,7-diazapyrenium with azidine in the presence of collidine and collidinium chloride.

6. Process for preparing a diazapyrene compound of formula :

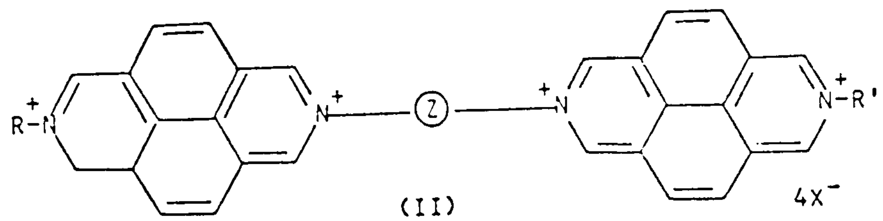

(II)

in which R and R' are identical or different and represent each hydrogen or an alkyl group with 1 to 6 carbon atoms; $X^-$ is an anion and Z is selected among the divalent radicals of formulae :

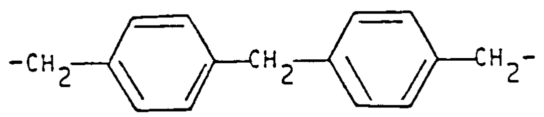

;

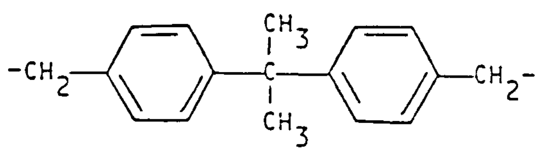

;

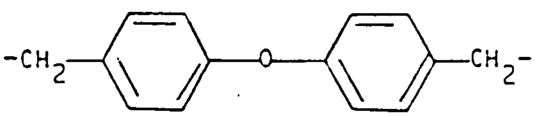

characterized in that it consists in reacting two molecules of 2,7-diazapyrene (DAP) with a molecule of a dihalogenide of formula $X_1$-Z-$X_2$, in which Z is as defined in claim 4, $X_1$ and $X_2$, identical or different, have the same signification as X, and then in carrying out the N-substitution of the nitrogens remaining in the DAP molecules according to the following reaction diagram :

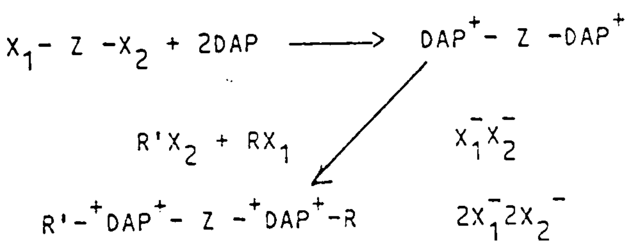


7. Process for the photocleavage of nucleic acids, characterized in that it consists in reacting, in the presence or in the absence of air (oxygen) or EDTA, an aqueous solution of a nucleic acid with a solution of a diazapyrene derivative, as obtained by the processes according to any one of claims 1 to 6 and in subjecting the resulting reaction mixture to irradiation with visible light.

**Patentansprüche**
**Patentansprüche für folgende Vertragsstaaten : AT, BE, CH, DE, FR, GB, GR, IT, LI, LU, NL, SE**

1. Diazapyren-Verbindung der Formel :

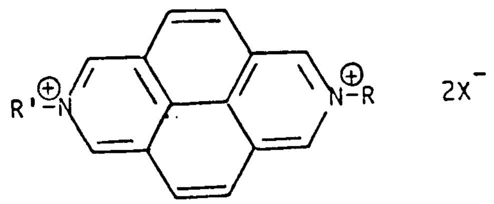

(I)

worin R' Wasserstoff oder eine Alkylgruppe mit 1 bis 6 Kohlenstoffatomen und R Wasserstoff oder eine substituierte, von den Gruppen der Formeln $-(CH_2)_n-SH$, $-(CH_2)_n-OH$ und $-(CH_2)_n-NH_2$ gewählte Alkylgruppe ist, in denen n zwischen 1 und 10 liegt, X ein Anion ist, unter der Bedingung, dass R und R' nicht gleichzeitig Wasserstoff sind.

**2.** N-Methyl-N'-Benzyl-2,7-Diazapyreniumdichlorid.

**3.** Diazapyren-Verbindung der Formel :

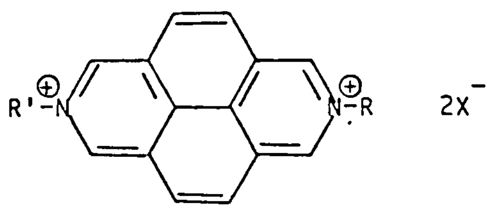

(I)

worin die beiden Gruppen R und R' identisch sind und (a) die Gruppe der Formel $-R_1Y-$ darstellen, worin $R_1$ $-(CH_2)_3-$ und $Y^-$ das Sulfonat-Ion $SO_3^-$ , ist oder (b) die Gruppe der Formel :

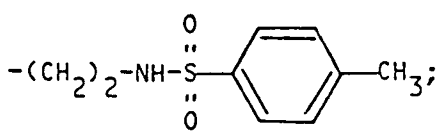

und

$X^-$ ein Anion ist.

**4.** Diazapyren-Verbindung der Formel :

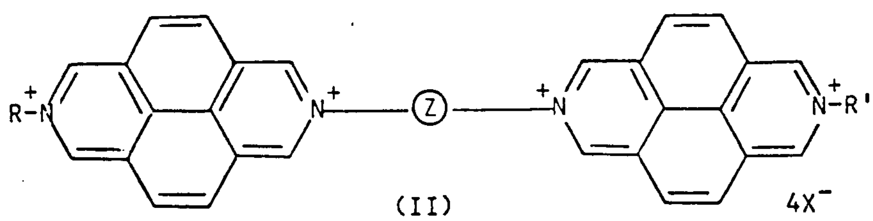

(II)

worin R und R' identisch oder verschieden sind, und jeweils Wasserstoff oder eine Alkylgruppe mit 1 bis 6 Kohlenstoffatomen darstellen; $X^-$ ist ein Anion und Z wird von den zweiwertigen Radikalen der Formeln :

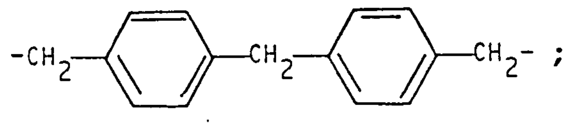

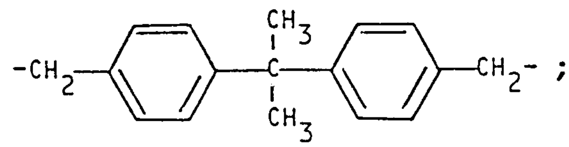

und

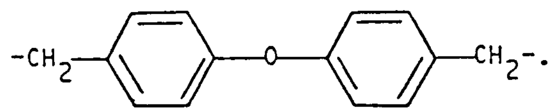

gewählt.

5. Diazapyren-Verbindung der Formel :

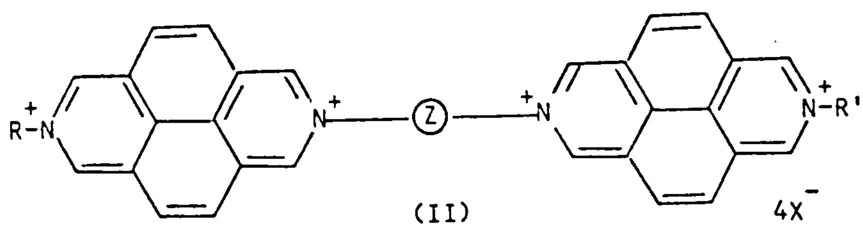

(II)

worin R und R' Methylgruppen sind, $X^-$ das Chlorion ist und Z $-CH_2-C_6H_4-CH_2-C_6H_4-CH_2-$ ist.

6. Verfahren zur photochemischen Spaltung von Nukleinsäuren, dadurch gekennzeichnet, dass es darin besteht, in der Gegenwart oder Abwesenheit von Luft (Sauerstoff) oder von EDTA, eine wässerige Lösung einer Nukleinsäure mit einer Lösung eines Diazapyren-Derivats, wie in einem der Ansprüche 1 bis 5 definiert, reagieren zu lassen und die erhaltene Reaktionsmischung einer Bestrahlung bei sichtbarem Licht auszusetzen.

7. Verfahren zur Herstellung einer Diazapyren-Verbindung nach Anspruch 1, dadurch gekennzeichnet, dass es im wesentlichen aus zwei Verfahrensschritten für die N-Substitution des 2,7-Diazapyrenium oder DAP nach den folgenden Reaktionsschemen :

$$DAP + RX_1 \rightarrow DAP^+\text{-}RX_1^-$$
$$DAP^+\text{-}RX_1^- + R'X_2 \rightarrow R^+\text{-}DAP^+\text{-}R'X_1X_2$$

besteht, worin $X_1$ und $X_2$, identisch oder verschieden, dieselbe Bedeutung haben wie X; und dass es eventuell darin besteht, die erhaltene Verbindung durch Ionenaustauch in die entsprechende Verbindung, in der $X_1$ und $X_2$ identisch sind, umzusetzen.

8. Verfahren zur Herstellung einer Diazapyren-Verbindung der Formel I nach Anspruch 1, in welcher R eine durch eine Hydroxygruppe substituierte Alkylgruppe ist und R' eine Alkylgruppe, dadurch gekennzeichnet, dass es darin besteht, zunächst in einer geeigneten Lösung durch Reaktion des 2,7-Diazapyrenium mit einem 2,4-Dinitrophenyl-Salz, wie Tosylat, ein 2-Dinitrophenyl-2,7-Diazapyrenium-Salz herzustellen, dann das genannte Zwischenprodukt mit einem durch ein R-Radikal substituierten

Amin reagieren zu lassen und dann eine N-Substitution mit einem Halogenid der Formel R'X nach Anspruch 7 durchzuführen.

**9.** Verfahren zur Herstellung einer Diazapyren-Verbindung der Formel I nach Anspruch 1, in welcher R eine durch eine Aminogruppe substituierte Alkylgruppe ist und R' eine Alkylgruppe, dadurch gekennzeichnet, dass es darin besteht, zunächst in einer geeigneten Lösung durch Reaktion des 2,7-Diazapyrenium mit einem 2,4-Dinitrophenyl-Salz, wie Tosylat, ein 2-Dinitrophenyl-2,7-Diazapyrenium-Salz herzustellen, dann das genannte Zwischenprodukt mit einem Diamin, von dem die eine Aminofunktion geschützt ist, die dann vor dem N-Substitutionsschritt mit einem Halogenid der Formel R'X nach Anspruch 7 entschützt wird.

**10.** Verfahren zur Herstellung einer Diazapyren-Verbindung der Formel I nach Anspruch 3, in der R und R' identisch sind und ein Radikal $R_1Y^-$ darstellt, wie in Anspruch 1 definiert, dadurch gekennzeichnet, dass es darin besteht, in wasserfreiem Aceton teilweise gelöstes 2,7-Diazapyrenium (DAP) und Propansulton bei der Rückflusstemperatur zusammen reagieren zu lassen.

**11.** Verfahren zur Herstellung eines Diazapyren-Derivates der Formel I nach Anspruch 3, in der R und R' identisch sind und die Gruppe der Formel :

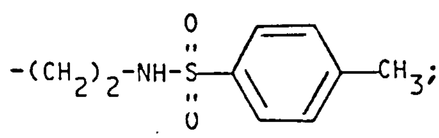

darstellen, dadurch gekennzeichnet, dass es darin besteht, 2,7-Diazapyrenium in Gegenwart von Kollidin und Kollidiniumchlorid mit Azidin reagieren zu lassen.

**12.** Verfahren zur Herstellung eines Diazapyren-Derivates nach Anspruch 4, dadurch gekennzeichnet, dass es darin besteht, zwei 2,7-Diazapyrenium (DAP) Moleküle mit einem Dihalogenid Molekül der Formel $X_1$-Z-$X_2$ reagieren zu lassen, in der Z wie in Anspruch 4 definiert ist, $X_1$ und $X_2$, identisch oder verschieden, dieselbe Bedeutung haben wie X, und dann die N-Substitution der in den DAP Molekülen verbleibenden Stickstoffe nach dem folgenden Reaktionsschema vorzunehmen :

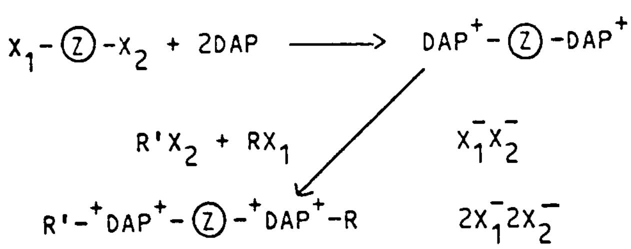

**Patentanspruch für folgenden Vertragsstaat : ES**

**1.** Verfahren zur Herstellung von Diazapyren-Derivaten der Formel :

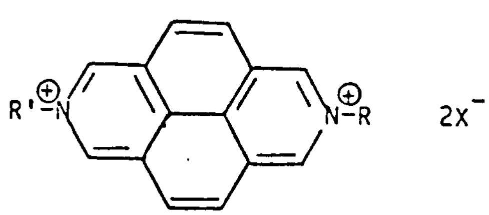

(I)

worin R' Wasserstoff oder eine Alkylgruppe mit 1 bis 6 Kohlenstoffatomen und R Wasserstoff oder eine substituierte, von den Gruppen der Formeln $-(CH_2)_n-SH$, $-(CH_2)_n-OH$ und $-(CH_2)_n-NH_2$ gewählte Alkyl-

gruppe ist, in denen n zwischen 1 und 10 liegt, X ein Anion ist, unter der Bedingung, dass R und R' nicht gleichzeitig Wasserstoff sind, dadurch gekennzeichnet, dass es im wesentlichen aus zwei Verfahrensschritten für die N-Substitution des 2,7-Diazapyrenium oder DAP nach den folgenden Reaktionsschemen:

$DAP + RX_1 \rightarrow DAP^+\text{-}RX_1^-$
$DAP^+\text{-}Rx_1^- + R'X_2 \rightarrow R^+\text{-}DAP^+\text{-}R'X_1X_2$

besteht, worin $X_1$ und $X_2$, identisch oder verschieden, dieselbe Bedeutung haben wie X; und dass es eventuell darin besteht, die erhaltene Verbindung durch Ionenaustauch in die entsprechende Verbindung, in der $X_1$ und $X_2$ identisch sind, umzusetzen.

2. Verfahren zur Herstellung einer Diazapyren-Verbindung der Formel I nach Anspruch 1, in welcher R eine durch eine Hydroxygruppe substituierte Alkylgruppe ist und R' eine Alkylgruppe, dadurch gekennzeichnet, dass es darin besteht, zunächst in einer geeigneten Lösung durch Reaktion des 2,7-Diazapyrenium mit einem 2,4-Dinitrophenyl-Salz, wie Tosylat, ein 2-Dinitrophenyl-2,7-Diazapyrenium-Salz herzustellen, dann das genannte Zwischenprodukt mit einem durch ein R-Radikal substituierten Amin reagieren zu lassen und dann eine N-Substitution mit einem Halogenid der Formel R'X nach Anspruch 1 durchzuführen.

3. Verfahren zur Herstellung einer Diazapyren-Verbindung der Formel I nach Anspruch 1, in welcher R eine durch eine Aminogruppe substituierte Alkylgruppe ist und R' eine Alkylgruppe, dadurch gekennzeichnet, dass es darin besteht, zunächst in einer geeigneten Lösung durch Reaktion des 2,7-Diazapyrenium mit einem 2,4-Dinitrophenyl-Salz, wie Tosylat, ein 2-Dinitrophenyl-2,7-Diazapyrenium-Salz herzustellen, dann das genannte Zwischenprodukt mit einem Diamin, von dem die eine Aminofunktion geschützt ist, die dann vor dem N-Substitutionsschritt mit einem Halogenid der Formel R'X nach Anspruch 1 entschützt wird.

4. Verfahren zur Herstellung einer Diazapyren-Verbindung der Formel

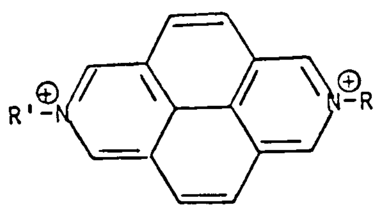

2X⁻ (I)

worin die beiden Gruppen R und R' identisch sind und die Gruppe der Formel $-R_1Y-$ darstellen, worin $R_1$ $-(CH_2)_3-$ ist und $Y^-$ das Sulfonat-Ion $SO_3^-$ , und $X^-$ ist ein Anion, dadurch gekennzeichnet, dass es darin besteht, in wasserfreiem Aceton teilweise gelöstes 2,7-Diazapyrenium (DAP) und Propansulton bei der Rückflusstemperatur zusammen reagieren zu lassen.

5. Verfahren zur Herstellung einer Diazapyren-Verbindung nach der Formel

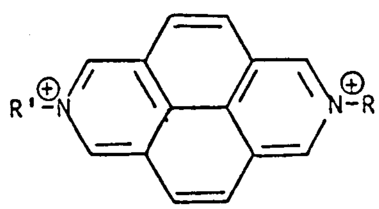

2X⁻ (I)

in welcher die beiden Gruppen R und R' identisch sind und die Gruppe der Formel

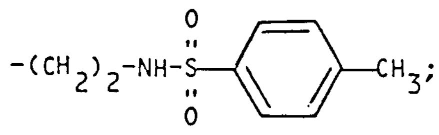

darstellen; und $X^-$ ist ein Anion, dadurch gekennzeichnet, dass es darin besteht, 2,7-Diazapyrenium in Gegenwart von Kollidin und Kollidiniumchlorid mit Azidin reagieren zu lassen.

**6.** Verfahren zur Herstellung einer Diazapyren-Verbindung der Formel :

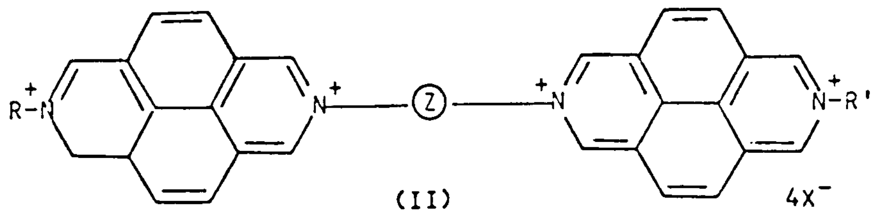

(II)

worin R und R' identisch oder verschieden sind, und jeweils Wasserstoff oder eine Alkylgruppe mit 1 bis 6 Kohlenstoffatomen darstellen; $X^-$ ist ein Anion und Z wird von den zweiwertigen Radikalen der Formeln :

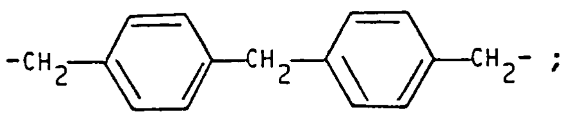

;

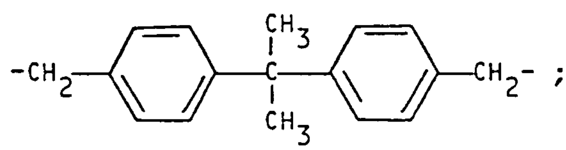

; und

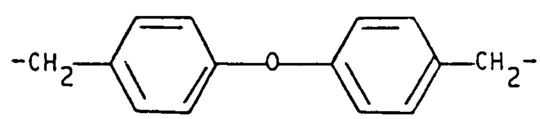

gewählt, dadurch gekennzeichnet, dass es darin besteht zwei 2,7-Diazapyrenium (DAP) Moleküle mit einem Dihalogenid Molekül der Formel $X_1$-Z-$X_2$ reagieren zu lassen, in der Z wie in Anspruch 4 definiert ist, $X_1$ und $X_2$, identisch oder verschieden, dieselbe Bedeutung haben wie X, und dann die N-Substitution der in den DAP Molekülen verbleibenden Stickstoffe nach dem folgenden Reaktionsschema vorzunehmen :

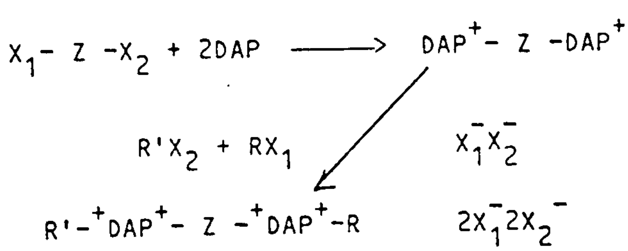

**7.** Verfahren zur photochemischen Spaltung von Nukleinsäuren, dadurch gekennzeichnet, dass es darin besteht, in der Gegenwart oder Abwesenheit von Luft (Sauerstoff) oder von EDTA, eine wässerige Lösung einer Nukleinsäure mit einer Lösung eines Diazapyren-Derivats, wie es durch die Verfahren nach einem der Ansprüche 1 bis 6 erhalten wird, reagieren zu lassen und die erhaltene Reaktionsmischung einer Bestrahlung bei sichtbarem Licht auszusetzen.

Fig.1

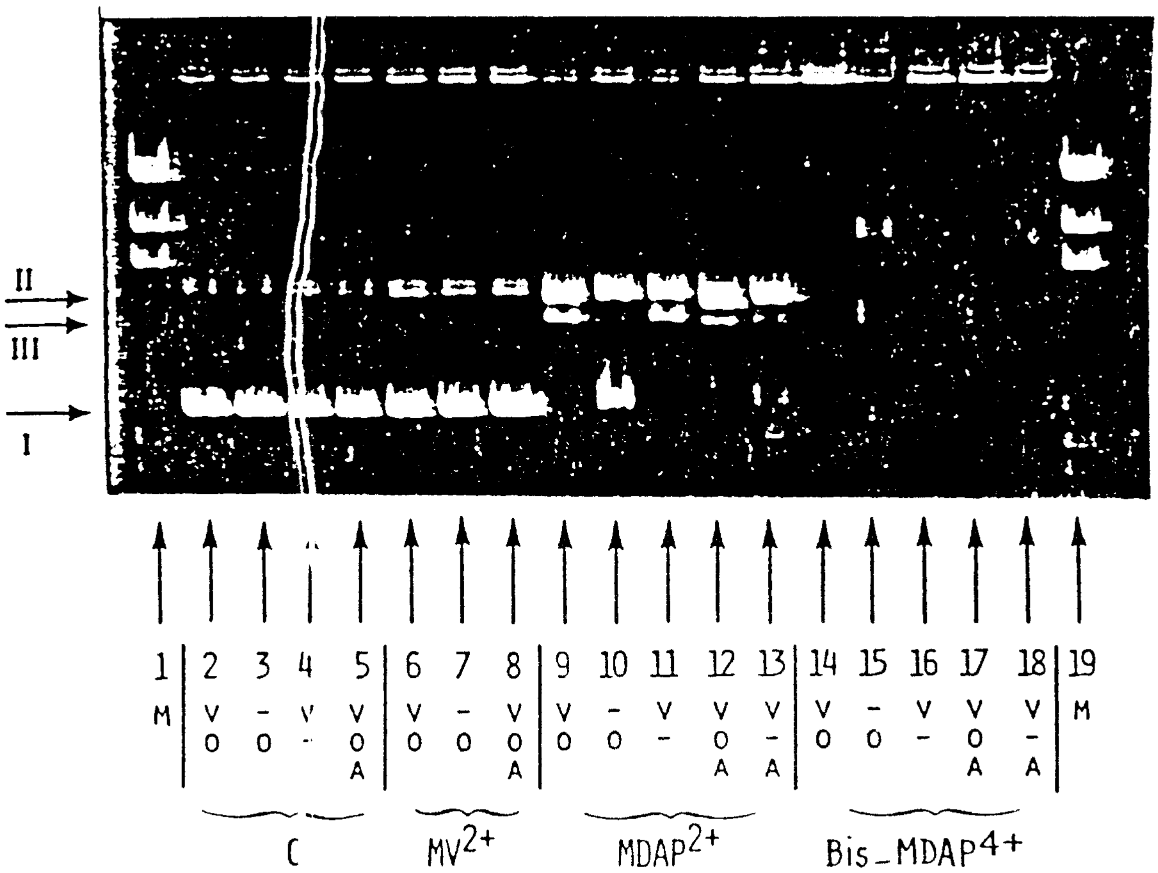
II
III
I
1 M
2 V O
3 - O
4 V -
5 V O A
6 V O
7 - O
8 V O A
9 V O
10 - O
11 V -
12 V O A
13 V - A
14 V O
15 - O
16 V -
17 V O A
18 V - A
19 M
C
MV2+
MDAP2+
Bis-MDAP4+

Fig-2